# EUROPEAN PATENT SPECIFICATION

(11) **EP 2 405 833 B1**
(45) Date of publication and mention of the grant of the patent: **14.12.2016**
(21) Application number: 10746936.3
(22) Date of filing: 26.02.2010
(51) Int. Cl.: A61B 17/58

(54) **SPINAL FIXATION ELEMENT ROTATION INSTRUMENT**
SPINALES FIXATIONSELEMENT MIT ROTATIONSINSTRUMENT
INSTRUMENT DE ROTATION D'UN ÉLÉMENT DE FIXATION VERTÉBRALE

(30) Priority: 27.02.2009 US 395469
(43) Date of publication of application: 18.01.2012
(73) Proprietor: DePuy Spine, Inc., Raynham, MA 02767-0350 (US)
(72) Inventor: STAD, Shawn, D., Fall River MA 02720 (US); RAMSAY, Christopher, L., West Wareham MA 02576 (US); GAMACHE, Thomas, Westport MA 02790 (US); MURPHY, Raymond, F., Attleboro MA 02703 (US)
(74) Representative: Orr, Robert
(86) International application number: PCT/US2010/025643
(87) International publication number: WO 2010/099478

(56) References cited:
- US-A- 4 507 990
- US-A- 4 507 990
- US-A- 5 329 834
- US-A1- 2003 208 203
- US-A1- 2005 038 432
- US-A1- 2008 243 190
- US-A1- 2008 255 563
- US-A1- 2008 312 703

## Description

### FIELD OF THE INVENTION

The present invention relates to spinal connection devices used in orthopedic surgery. More particularly, the present invention may relate to a spinal fixation element rotation instrument with dual lever arms that couple to and rotate a spinal fixation element when positioning the spinal fixation element through bone anchors placed on vertebrae.

### BACKGROUND OF THE INVENTION

Spinal fixation systems may be used in surgery to align, adjust and/or fix portions of the spinal column, i.e., vertebrae, in a desired spatial relationship relative to each other. Many spinal fixation systems employ a spinal fixation element, e.g. a spinal fixation rod, for supporting the spine and for properly positioning components of the spine for various treatment purposes. Vertebral bone anchors, comprising pins, bolts, screws, and hooks, engage the vertebrae and connect the supporting spinal fixation element to different vertebrae. The size, length and shape of the spinal fixation element depend on the size, number and position of the vertebrae to be held in a desired spatial relationship relative to each other by the apparatus.

Spinal fixation elements can be anchored to specific portions of the vertebra. Since each vertebra varies in shape and size, a variety of anchoring devices have been developed to facilitate engagement of a particular portion of the bone. Pedicle screw assemblies, for example, have a shape and size that is configured to engage pedicle bone. Such screws typically include a threaded shank that is adapted to be threaded into a vertebra, and a head portion having a spinal fixation element-receiving portion for receiving a spinal fixation element. A set-screw, plug, cap or similar type of closure mechanism is used to lock the spinal fixation element into the spinal fixation element-receiving portion of the pedicle screw.

In conventional spinal surgery, first, anchoring devices are attached to vertebra, then a spinal rod is aligned with the anchoring devices and secured. For example, for conventional pedicle screw assemblies, first the engagement portion of each pedicle screw is threaded into a vertebra. Once the pedicle screw assembly is properly positioned, a spinal fixation rod is seated in the rod-receiving portion of each pedicle screw head. The rod is locked into place by tightening a cap or similar type of closure mechanism to securely interconnect each pedicle screw to the fixation rod. This type of conventional spinal surgical technique usually involves making a surgical access opening in the back of the patient. Because exact placement of the screw assemblies depends on a patient's particular bone structure and bone quality, the exact position of all screw assemblies cannot be known until after all the assemblies are positioned. Adjustments, such as bending, are made to the spinal rod to ensure that it aligns with each screw assembly.

When placing the spinal fixation element in a long construct, the spinal fixation element may be inserted in an inverted orientation to facilitate insertion and positioning of the spinal fixation element below fascia. Consequently, the spinal fixation element needs to be rotated prior to final positioning in order to match the curvature of the spine. The contemporary medical devices, such as spinal fixation element holders, do not accommodate the rotation of the spinal fixation element while performing a minimally invasive procedure. Rotating the spinal fixation element percutaneously typically requires an additional skin incision or increasing the size of the existing skin incision. Additionally, it is desirable to make minimal rotary adjustments for better control. Currently, surgeons have to select a long rod to provide a holding surface when the rod is placed through the skin incision. The extra portion of the rod is cut in situ, extending the surgery time and posing the risk of cutting the rod at an unintentional location.

The contemporary medical devices also require re-engagement of the device to the spinal fixation element multiple times and often a second instrument is required during the re-engagement to prevent the spinal fixation element from slipping back toward the initial position. Therefore, there is a need for an instrument that will accommodate the rotation of a spinal fixation element while preventing the spinal fixation element from rotating back toward the initial position following adjustment during a minimally invasive procedure.

US Patent Publication No.US-2008/0312703 discloses instrumentation for delivering and installing components such as a spine rod, set screw and other required hardware to vertebral anchors. One disclosed instrument is a spine rod installation tool which includes an elongate member having a proximal end spaced from a distal end and a handle located at the proximal end of the elongate member for manipulation of the tool. A second elongate member has a proximal end spaced from a distal end and a handle located at the proximal end. The two elongate members are pivotally coupled together by a pin located between the proximal and distal ends. The installation tool is a scissor-like instrument in which movement of the proximal ends of the members toward and away from each other likewise moves the distal ends of the members toward and away from each other, to insert a spine rod through channels in heads of vertebral anchors.

US Patent Publication No.US-2003/0208203 discloses instruments for inserting one or more implants to a surgical site in a patient. The implant is mountable to the instrument in a reduced profile orientation and after insertion is manipulated with the insertion instrument to the desired orientation. One disclosed insertion instrument includes a handle assembly, a shaft assembly and an implant holder at the distal end of the shaft assembly. The implant holder is configured to clamp or grip surfaces of the implant, which takes the form of a spinal rod. The rod is releasably mounted to the insertion instrument with the implant holder, and is positioned on the insertion instrument in a first position having a reduced profile orientation in which a longitudinal axis of the rod extends at an angle to a longitudinal axis of the shaft assembly. After insertion, the rod is moved from its low profile insertion orientation to an enlarged profile orientation that allows it to be coupled to anchors such as bone screws with an implant receptacle. The handle assembly is manipulated to actuate the implant holder through the shaft assembly to the enlarged profile orientation. In the second, enlarged profile orientation, the longitudinal axis of the rod extends transversely with respect to the longitudinal axis of the shaft assembly and also transversely to the direction of insertion of the rod.

US Patent No.US-4507990 discloses a ratchet wrench having a top housing and a first handle cooperating with the top housing; a bottom housing with a cooperating second handle; a ratchet drum disposed within the top housing and the bottom housing and provided with exterior teeth and a pair of side bevel gears rotatably mounted on a shaft in opposed relationship inside the ratchet drum; a top bevel gear disposed inside the top housing and extending inside the ratchet drum to engage the side bevel gears; a drive bevel gear rotatably positioned in the bottom housing opposite the top bevel gear and also engaging the side bevel gears and carrying a socket drive extending from the bottom housing; top and bottom ratchet means mounted on the first and second handles, respectively and selectively cooperating with the teeth on the ratchet drum; and pivoting handle lock means for selectively allowing the first and second handles to be manipulated as one handle or separately.

### SUMMARY

Embodiments of the present invention provide a spinal fixation element rotation instrument that allows controlled rotation of spinal fixation elements. The spinal fixation element rotation instrument includes two lever arms connected to each other at a distal end thereof. The first arm, i.e. the holder, is held stationary while the second arm, i.e. the driver, is configured to rotate in a predetermined direction relative to the first arm. The driver rotates the spinal fixation element within the bone anchors or the extensions thereof while the holder prevents the spinal fixation element from rotating back toward its initial position. The spinal fixation element rotation instrument rotates the spinal fixation element to steer the spinal fixation element through one or more bone anchors attached to vertebrae. The spinal fixation element rotation instrument may also force the bone anchors to move the vertebrae attached thereto to bring the spine into alignment.

According to a first aspect of the invention, an instrument for rotating a spinal fixation element is provided. The instrument includes a first lever arm and a second lever arm. A distal end of the first lever arm is adapted to couple to the spinal fixation element. The second lever arm is rotatably coupled to the first lever arm at the distal end thereof. A distal end of the second lever arm is adapted to couple to the spinal fixation element. The second lever arm rotates relative to the first lever arm, about a central axis of the distal ends of the first lever arm and the second lever arm to rotate the spinal fixation element from an initial position to a rotated position.

According to various aspects of the present invention, the first lever arm includes a first ratchet and the second lever arm includes a second ratchet forming a dual ratchet mechanism that prevents the spinal fixation element from rotating back toward the initial position. The instrument further includes an extension element. The extension element has a distal end adapted to couple to the spinal fixation element and a proximal end adapted to couple to the instrument. A portion of the extension element fits through a skin incision at a target site to guide the spinal fixation element under skin.

According to another aspect, the second lever arm may further include a first section, a second section and an attachment mechanism. The attachment mechanism attaches the first section to the second section so that the first section is rotatable relative to the second section about a central axis of the attachment mechanism. The central axis of the attachment mechanism is perpendicular to the central axis of the distal ends of the first lever arm and the second lever arm.

According to yet another aspect, the instrument may also include a first switch provided on a proximal end of the first lever arm. The first switch, when positioned at a first position, allows the first ratchet to rotate clockwise. The first switch, when positioned at a second position, allows the first ratchet to rotate counterclockwise. The instrument may also include a second switch provided on a proximal end of the second lever arm. The second switch, when positioned at a first position, allows the second ratchet to rotate clockwise. The second switch, when positioned at a second position, allows the second ratchet to rotate counterclockwise. The first ratchet and the second ratchet lock each other when the first switch is at the first position and the second switch is at the second position. At the locked out position, the first ratchet and the second ratchet prevent the spinal fixation element from rotating.

According to another aspect, a method, not claimed, for percutaneous positioning of a spinal fixation element through a plurality of bone anchors placed below fascia is presented. The plurality of bone anchors have an opening adapted to couple to the spinal fixation element. A proximal end of the spinal fixation element is coupled to a spinal fixation element rotation instrument including two lever arms connected to each other at a distal end of the spinal fixation element rotation instrument. The second lever arm rotates relative to a first lever arm about a central axis of the distal end of the spinal fixation element rotation instrument. A distal end of the spinal fixation element is inserted through an incision in proximity of a first bone anchor. The spinal fixation element is guided through the opening of the first bone anchor and a second bone anchor adjacent to the first bone anchor. The spinal fixation element is rotated within the distal end of the spinal fixation element rotation instrument using the spinal fixation element rotation instrument to accommodate the bone anchors.

According to another aspect of the present invention, a dual ratchet spinal fixation element rotation instrument holder is provided. The dual ratchet spinal fixation element rotation instrument includes a first ratchet and a second ratchet connected to the first ratchet at a distal end thereof. The first ratchet and the second ratchet are configured to couple to a spinal fixation element or to an extension element coupled to the spinal fixation element. One of the first ratchet or the second ratchet rotates the spinal fixation element in a first direction from an initial position to a rotated position. Other of the first ratchet or the second ratchet prevents the spinal fixation element from rotating back toward the initial position.

According to yet another aspect, a method, not claimed, for positioning of a spinal fixation element through a plurality of bone anchors placed below fascia is provided. The plurality of bone anchors have an opening adapted to couple to the spinal fixation element. A proximal end of the spinal fixation element is coupled to a spinal fixation element rotation instrument having a lever arm. The spinal fixation element is placed through the opening of the first bone anchor. The spinal fixation element is guided though a second bone anchor. The lever arm is rotated in a first direction to effect rotation of the spinal fixation element about a central axis of the spinal fixation element in the first direction to a rotational position. The lever arm is rotated in a second direction, opposite the first direction, while maintaining the spinal fixation element in the rotational position.

### BRIEF DESCRIPTION OF THE FIGURES

The foregoing and other objects, features and advantages of the invention will be apparent from the following description and apparent from the accompanying drawings, in which like reference characters refer to the same parts throughout the different views. The drawings illustrate principles of the invention and, although not to scale, show relative dimensions.
**Figures 1A** **and** **1B** illustrate an exemplary spinal fixation element rotation instrument, an exemplary extension element and an exemplary spinal fixation element;
**Figure 2A** illustrates a cross-sectional view of an exemplary extension element coupled to an exemplary spinal fixation element rotation instrument;
**Figure 2B** illustrates a cross-sectional view of a carrier of the exemplary extension element illustrated in **Figure 2A****;**
**Figure 2C** illustrates a cross-sectional view of the distal end of the exemplary spinal fixation element rotation instrument illustrated in **Figure 2A**;
**Figures 3A and 3B** illustrate exemplary spinal fixation elements according to various embodiments of the present invention;
**Figures 4A-4C** illustrate an exemplary coupling mechanism that couples the spinal fixation element to the extension element;
**Figures 5A and 5B** illustrate another exemplary coupling mechanism that couples the spinal fixation element to the extension element;
**Figures 6A and 6B** illustrate a first switch provided on the first lever arm and a second switch provided on the second lever arm;
**Figures 6C and 6D** illustrate a first ratchet and a second ratchet provided at a distal end of the spinal fixation element rotation instrument according to an exemplary embodiment of the present invention;
**Figure 7** illustrates an exemplary spinal fixation element rotation instrument coupled to the exemplary extension element that is coupled to the exemplary spinal fixation element;
**Figures 8A** **and** **8B** illustrate an exemplary configuration of a second lever arm including a first section and a second section;
**Figures 9A** **and** **9B** illustrate the second section of the second lever arm at an angled position relative to the first section of the second lever arm;
**Figures 10A** **and** **10B** illustrate the placement of the spinal fixation element through a plurality of bone anchors using the spinal fixation element rotation instrument;
**Figures 11A** **and** **11B** are a flow diagram, not claimed, of placing a spinal fixation element through a plurality of bone anchors;
**Figure 12** illustrates an exemplary spinal fixation element according to another embodiment of the present invention;
**Figure 13** illustrates an exploded view of the exemplary spinal fixation element rotation instrument illustrated in **Figure 12****;**
**Figures 14A-14D** illustrate the locked position of the exemplary spinal fixation element rotation instrument illustrated in **Figures 12** **and** **13****;**
**Figures 15A-15C** illustrate the unlocked position of the exemplary spinal fixation element rotation instrument illustrated in **Figures 12** **and** **13****;**
**Figures 16A-16C** illustrates the ratchet mechanism of the exemplary spinal fixation element rotation instrument illustrated in **Figures 12** **and** **13****;**
**Figure 17** illustrates the exemplary spinal fixation element rotation instrument illustrated in **Figures 12** **and** **13****,** which is coupled to an exemplary extension element coupled to an exemplary spinal fixation element;
**Figure 18** illustrates a cross-sectional view of the exemplary spinal fixation element rotation instrument, the exemplary extension element and the exemplary spinal fixation element illustrated in **Figure 17****;**
**Figures 19A and 19B** illustrate another cross-sectional view of the exemplary spinal fixation element rotation instrument, the exemplary extension element and the exemplary spinal fixation element illustrated in **Figure 17****;** and
**Figure 20** illustrates another configuration of the exemplary spinal fixation element rotation instrument, the exemplary extension element and the exemplary spinal fixation element illustrated in **Figure 17****.**

### DETAILED DESCRIPTION OF THE INVENTION

Exemplary embodiments of the present invention provide an improved spinal fixation element rotation instrument to be used in placing a spinal fixation element through a plurality of vertebral bone anchors during a minimally invasive surgery for correcting a deformity or a degenerative spine disorder. One of ordinary skill in the art will recognize that the present invention is not limited to use in spinal surgery, and that the instrument described herein can be adapted for use with any suitable surgical device to be placed in a desired position in a variety of medical procedures.

The spinal fixation element rotation instrument of some of the exemplary embodiments has two lever arms that are connected to each other at distal ends thereof. Each lever arm rotates relative to the other lever arm. The distal ends of the first and second lever arms are adapted to couple to a spinal fixation element or an extension element that couples to the spinal fixation element. The spinal fixation element is secured within the distal ends of the first and second lever arms. The distal ends of the lever arms may have a dual ratchet feature that prevents rotation of the spinal fixation element in a set direction. When one arm, i.e. the driver, rotates back and forth, the other arm, i.e. the holder, is held stationary. As a result, the spinal fixation element rotates in a predetermined direction from an initial position to a rotated position and is prevented from rotating back toward the initial position.

The rotational direction of the lever arms, thus the rotational direction of the spinal fixation element, may be set using knobs or switches provided at a proximal end of one or both of the lever arms. Setting the switch of the driver arm to a first position may allow the driver arm to rotate the spinal fixation element clockwise. Similarly, setting the switch of the driver arm to a second position may allow the driver arm to rotate the spinal fixation element counterclockwise.

According to various embodiments of the present invention, the switches provided on the first and second lever arms may be set in a position that locks the rotation of the lever arms. In the locked out position, the lever arms no longer have the ratcheting feature and the spinal extension element is prevented from rotating within the distal ends of the lever arms. Therefore, if the user would like to change the rotational direction of the spinal fixation element, the user would have to change the rotational direction of one of the driver arm or the holder arm. If the user only changes the rotational direction of both of the driver arm and the holder arm, the instrument will be in the locked out position.

The driver arm of the spinal fixation element rotation instrument may be provided with two sections attached to each other via a hinge or a loaded spring mechanism. The hinge may allow the first section to rotate about a central axis of the hinge, relative to the second section. This way, the first section may be moved away from the screw extensions providing clearance during spinal fixation element rotation and enabling the user to relocate the first section to better suit the conditions of the procedure.

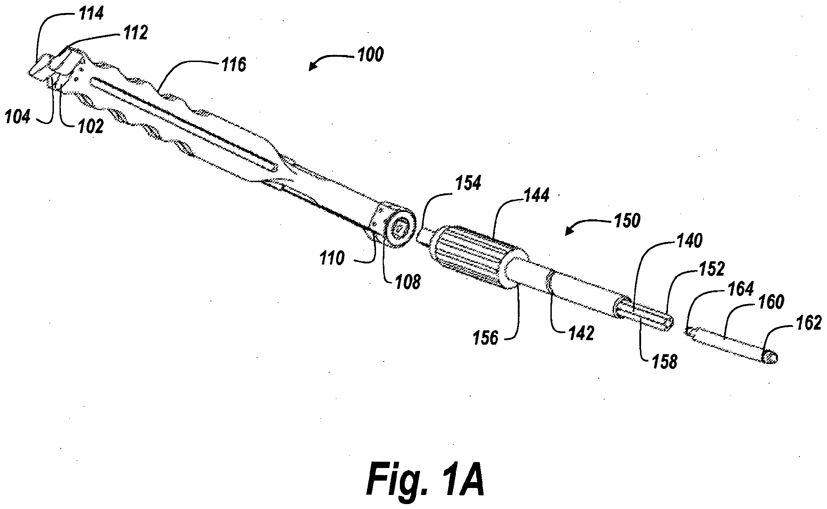

**Figure 1A** illustrates a profile view of an exemplary spinal fixation element rotation instrument 100, an exemplary extension element 150 and an exemplary spinal fixation element 160. A proximal end 154 of the extension element 150 couples to the spinal fixation element rotation instrument 100 and a distal end 152 of the extension element 150 couples to the spinal fixation element 160.

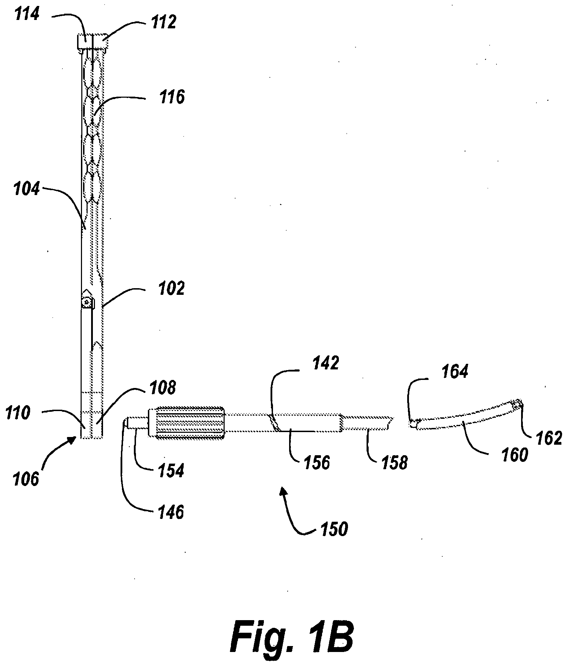

As illustrated in **Figures 1A** **and** **1B****,** the extension element 150 includes a sleeve 156 provided around a carrier 158. The carrier 158 is adapted to couple to the spinal fixation element 160. The carrier 158 may include a coupling feature 140, such as a leaf spring, for tightly holding the spinal fixation element 160. The coupling feature 140 prevents inadvertent decoupling of the spinal fixation element 160 from the extension element 150. For example, the spinal fixation element 160 may be coupled to the extension element 150 via a spring loaded tooth. Alternatively, the coupling feature 140 may grab on the surface features provided at the proximal end 164 of the spinal fixation element 160. Exemplary coupling mechanisms coupling the extension element 150 and the spinal fixation element 160 according to various embodiments of the present invention will be described below.

The sleeve 156 may be slid over the carrier 158 upon the carrier 158 couples to the spinal fixation element 160. As such, the location where the carrier 158 engages the spinal fixation element 160 is protected by the sleeve 156 to prevent inadvertent decoupling of the spinal fixation element 160 from the carrier 158. In an exemplary embodiment of the present invention, the sleeve 156 may include a helical cut 142 and the carrier 158 may include a protrusion, such as a pin, that slides in the angular cut 142 of the sleeve 158. The pin and the helical cut 142 allow the sleeve 156 to move forward and backward over the carrier 158. The pin moves within the helical cut 142 when the user twists and slides the sleeve 156 over the carrier 158. The sleeve 156 may be provided with a grooved surface portion 144 to improve the user's grip on the extension element 150 and to facilitate rotation of the sleeve 156 over the carrier 158.

The spinal fixation element rotation instrument 100 illustrated in **Figures 1A** **and** **1B** is formed of two lever arms 102 and 104. The first lever arm 102 and the second lever arm 104 may be provided with surface features 116 for improved ergonomics and improved control over the spinal fixation element rotation instrument 100. A first ratchet 108 is provided at the distal end of the first lever arm 102. A second ratchet 110 is provided at the distal end of the second lever arm 104. The first ratchet 108 and the second ratchet 110 are coupled to each other. The proximal end 154 of the extension element 150 couples to the spinal fixation element rotation instrument 100 by fitting through the first ratchet 108 and the second ratchet 110. The rotation of the first ratchet 108 and/or the second ratchet 110 rotates the extension element 150 coupled to the spinal fixation element 160. The rotational direction of the spinal fixation element 160 depends on the rotational direction of the first ratchet 108 and/or the second ratchet 110. One of ordinary skill in the art will appreciate that the first ratchet 108 and the second ratchet 110 may couple to a variety of modular tips and that the modularity is not limited to the extension element 150.

The extension element 150 may be provided with a plurality of markers to illustrate how much the extension element 150 has rotated within the distal end 106 of the spinal fixation element rotation instrument 100.

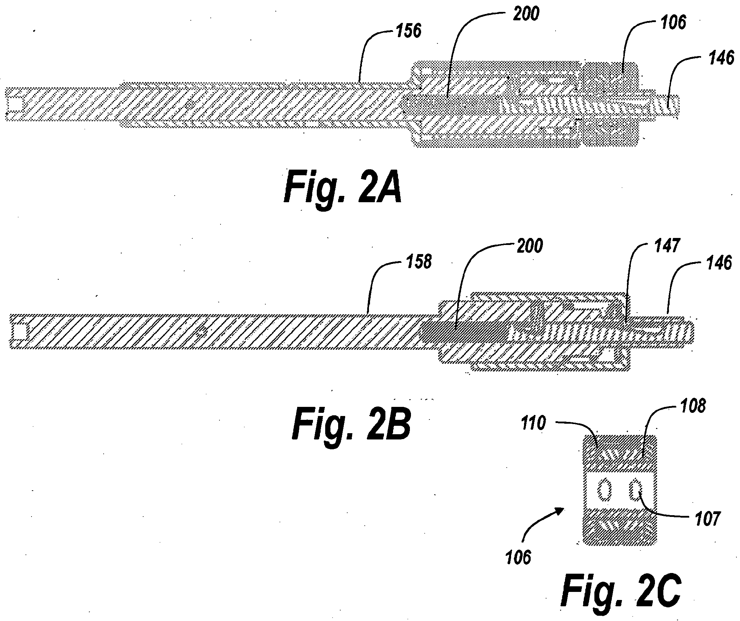

The extension element 150 may also include a coupling/decoupling mechanism 146 for coupling and decoupling the extension element 150 to/from the spinal fixation element rotation instrument 100. **Figure 2A** illustrates a cross-sectional view of the extension element 150 coupled to the distal end of the spinal fixation element rotation instrument 100. **Figure 2B** illustrates a cross-sectional view of the extension element 150 and the decoupling mechanism 146 provided at a proximal end 154 of the extension element 150. **Figure 2C** illustrates a cross-sectional view of the distal end 106 of the spinal fixation element rotation instrument 100. The coupling/decoupling mechanism 146 may comprise a loaded spring 200 that is used to lock the extension element 150 in the distal end 106 of the spinal fixation element rotation instrument 100. By pressing on the coupling/decoupling mechanism 146, the user compresses the loaded spring 200. When the user places the extension element 150 through the distal end 106 of the spinal fixation element rotation instrument 100, the loaded spring 200 decompresses and pushes the ball 147 provided on a proximal end 154 of the extension element 150 outward. As illustrated in **Figure 2C****,** one or more grooves 107 may be provided in an internal surface of the first ratchet 108 and/or the second ratchet 110. The ball 147 falls into one of the grooves 107 provided on the internal surface of the distal end 106 of the spinal fixation element rotation instrument 100. When the ball 147 is in the groove 107, the extension element 150 is locked in the distal end 106 of the spinal fixation element rotation instrument 100. The user may press on the coupling/decoupling mechanism 154 to disengage the ball 147 from the groove 107, unlocking the extension element 150 from the spinal fixation element rotation instrument 100.

One of ordinary skill in the art will appreciate that the coupling/decoupling mechanism 146 described above is for illustrative purposes only and similar mechanisms may be used to couple the extension element 150 to the spinal fixation element rotation instrument 100. Moreover, the proximal end 154 of the extension element 150 may have a variety of shapes and sizes. The spinal fixation element rotation instrument 100 may be provided with an appropriate distal end 106 that matches the shape and size of the proximal end 154 of the extension element 150.

According to various embodiments of the present invention, additional modular tips may be provided to facilitate the coupling between the spinal fixation element rotation instrument 100 and the extension element 150. A first end of the modular tip couples to the distal end of the spinal fixation element rotation instrument 100 and a second end, opposite to the first end, couples to the proximal end of the extension element 150. The first and second ends of the modular tips may be, for example, circular, hexagonal, diamond shape, etc. according to the shape of the distal end 106 of the spinal fixation element rotation instrument 100 and the proximal end 154 of the extension element 150.

According to another exemple, the spinal fixation element rotation instrument 100 may directly couple to the spinal fixation element 160 eliminating the extension element 150. In this exemplary embodiment, the spinal fixation element 160 may fit through a portion of the first ratchet 108 and/or the second ratchet 110. The spinal fixation element 160 may have surface features such as threads or grooves that enable a portion of the first ratchet 108 and/or the second ratchet 110 to grab on the spinal fixation element 160. The spinal fixation element 160 may be of any shape and size, including but not limited to, hexagonal, square, round, diamond, etc.

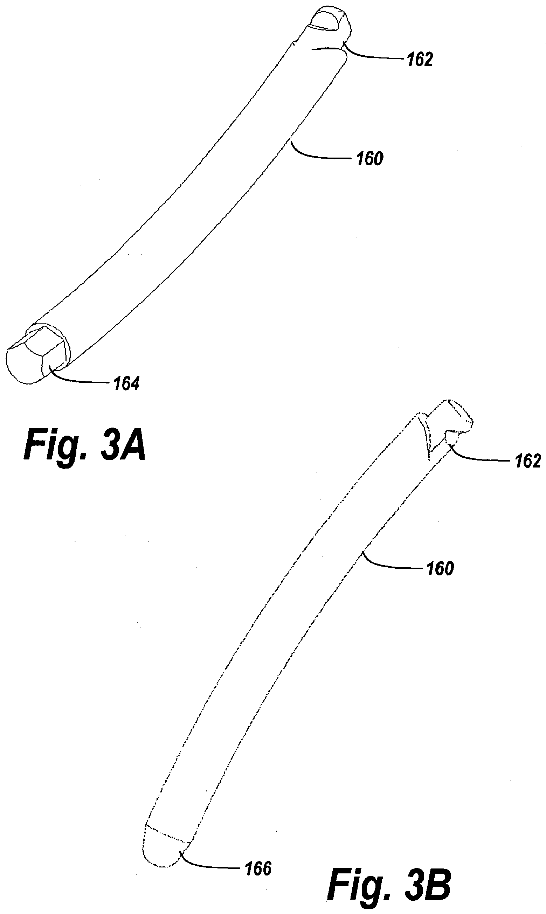

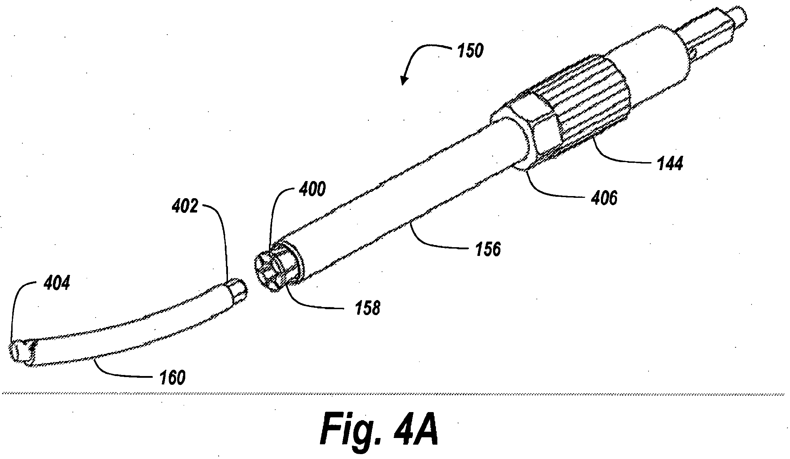

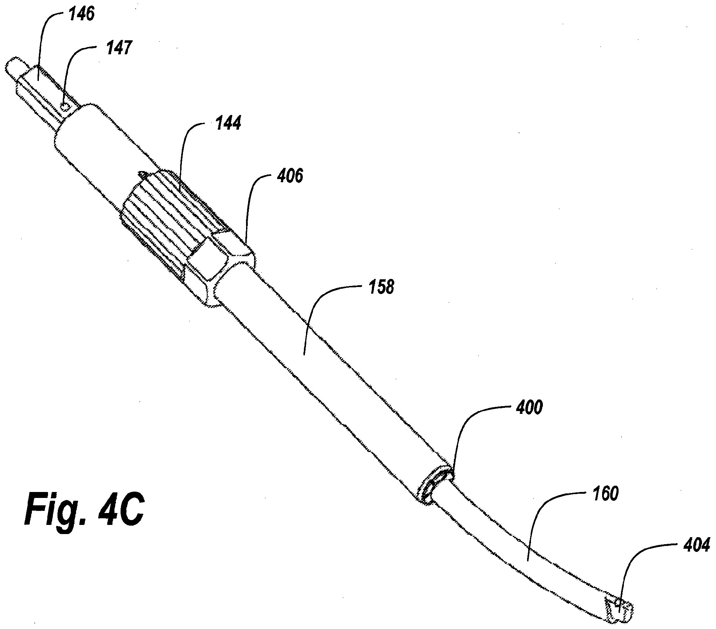

**Figures 3A and 3B** illustrate two exemplary spinal fixation elements 160 according to various embodiments of the present inventions. As illustrated in **Figure 3A**, the proximal end 164 of the spinal fixation element 160 may have a hexagonal shape. **Figure 3B** illustrates an embodiment of the present invention where the spinal fixation element 160 is provided with a rounded proximal end 166. The distal end 162 of the spinal fixation elements 160 illustrated in **Figures 3A** and **3B** may be of any shape. According to various embodiments of the present application, the spinal fixation elements, such as those illustrated in **Figure 3A****,** may also be used by reversing the distal end and the proximal end of the spinal fixation elements. **Figures 4A-4C** illustrate an exemplary embodiment where the distal end 164 of the spinal fixation element 160 illustrated in **Figure 3A** becomes the proximal end 402 and is coupled to the extension element 150. The proximal end 162 of the spinal fixation element 160 illustrated in **Figure 3A** becomes the distal end 404 and is inserted into the patient.

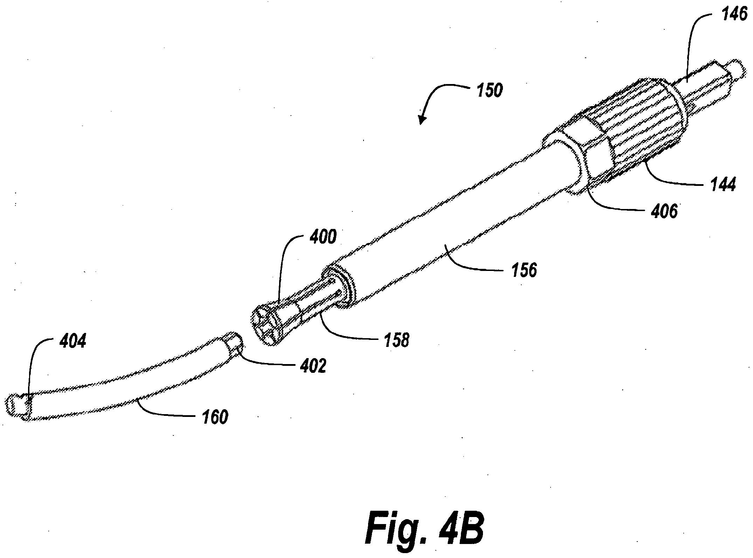

**Figures 4A-4C** illustrate an exemplary mechanism 400 to couple the spinal fixation element 160 to the extension element 150. As illustrated in **Figure 4A****,** the carrier 158 is provided with a grabbing tip 400. The grabbing tip 400 assumes a larger diameter when the sleeve 156 is retracted over the carrier 158, as illustrated in **Figure 4B****.** The proximale end 402 of the spinal fixation element 160 is inserted in the grabbing tip 400 when the sleeve 156 is in a retracted position. The sleeve 156 is then advanced over the carrier 158 and the grabbing tip 400 so as to cover and collapse the grabbing tip 400. When the sleeve 156 is advanced over the grabbing tip 400, the opening of the grabbing tip 400 closes over the proximal end 402 of the spinal fixation element 160 so as to tightly grab or collapse the spinal fixation element 160.

A portion of the exemplary sleeve 156 illustrated in **Figures 4A-4C** may be provided with a plurality of threads. The treads may be 5-10 mm. long, provided on a portion of the inner surface of the sleeve 156. The threads may engage a portion of the carrier 158. The threads may slowly disengage from the carrier 158 while the sleeve 156 is retracted over the carrier 158 to expose the grabbing tip 400. When the threads are completely disengaged from the carrier 158, the sleeve 156 may be pulled up to expose the remainder of the carrier 158. The threads may help the sleeve 156 to tighten or loosen up around the carrier 158 and the grabbing tip 400.

The grooved surface portion 144 of the sleeve 156 may provide improved control to the user over the sleeve 156. The user may rotate the sleeve 156 around the carrier 158 using the grooved surface portion 144 so as to advance or retract the sleeve 156 over the carrier 158. The extension element 150 illustrated in **Figures 4A-4C** may have a coupling/decoupling mechanism 146 similar to one discussed above to couple the extension element 150 to the spinal fixation element rotation instrument 100. The sleeve 156 may also include an external surface feature 406 for mating with a second instrument when the spinal fixation element 160 is in its final place and the extension element 150 needs to be removed. During removal of the extension element 150, it may be difficult for the surgeon to get their hand to a position where the surgeon can loosen the sleeve 156 and detach the spinal fixation element 160 from the extension element 150. If this is the case, the second instrument, e.g. a hex wrench that mates with the external surface feature 406 of the sleeve 156, may be used to grab on the sleeve 156 for rotating it to release the extension element 150 from the sleeve 156.

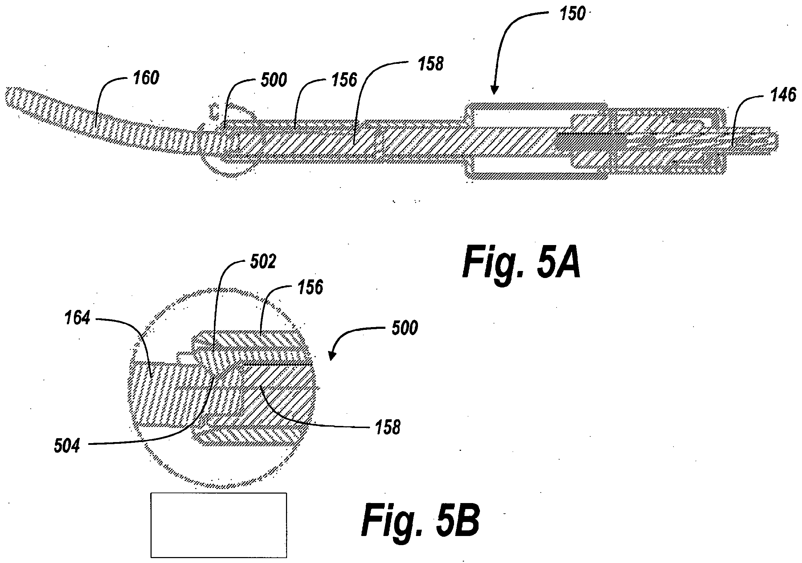

**Figures 5A and 5B** illustrate another exemplary mechanism 500 to couple the spinal fixation element 160 to the extension element 150. As illustrated in **Figure 5B**, the internal surface of the carrier 158 is provided with a leaf spring 502. The proximal end 164 of the spinal fixation element 160 is provided with a notch 504. When the spinal fixation element 160 is coupled to the extension element 150, the leaf spring 502 of the carrier 158 grabs the notch 504 provided on the proximal end 164 of the spinal fixation element 160. The coupling location of the spinal fixation element 160 and the extension element 150 is provided within the sleeve 156 to prevent unintentional decoupling of the spinal fixation element 160 from the extension element 150.

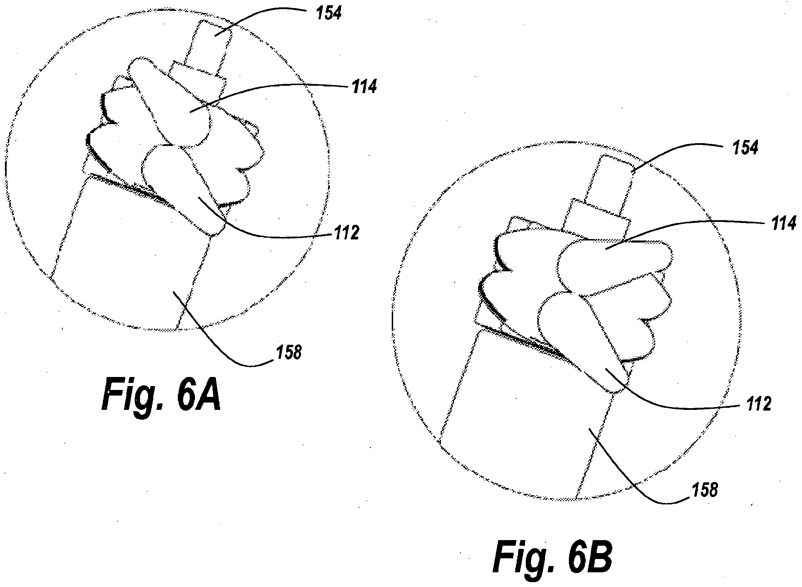

**Figures 6A and 6B** illustrate a top view of the spinal fixation element rotation instrument 100. The first lever arm 102 includes a switch or a knob 112 at the proximal end thereof. Similarly, the second lever arm 104 includes a switch or a knob 114 at the proximal end thereof. The switch 112 is used to adjust the rotational direction of the first ratchet 108. Similarly, the switch 114 is used to adjust the rotational direction of the second ratchet 110. Using the switch 112, the first ratchet 108 may be set to rotate clockwise or counterclockwise. Using the switch 114, the second ratchet 110 may be set to rotate clockwise or counterclockwise. The rotation of the first ratchet 108 or the rotation of the second ratchet 110 rotates the extension element 150 that is coupled to the spinal fixation element rotation instrument 100. Alternatively. the spinal fixation element 160 may be coupled to the spinal fixation element rotation instrument 100 without the extension element 150. In this case, the rotation of the first ratchet 108 or the rotation of the second ratchet 110 rotates the spinal fixation element 160 that is coupled to the spinal fixation element rotation instrument 100.

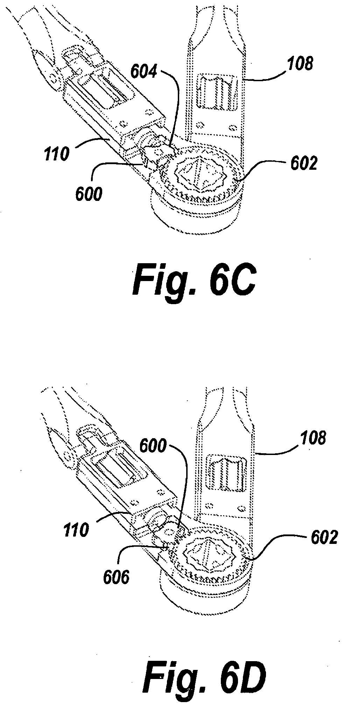

As shown in **Figure 6C****,** when the second ratchet 110 is set to rotate in a first direction, i.e. clockwise, a right end 604 of the rocker 600 engages the teeth 602 of the second ratchet 110. Clockwise rotation of the second ratchet 110 rotates the spinal fixation element 160 clockwise. As shown in **Figure 6D****,** when the second ratchet 110 is set to rotate counterclockwise, a left end 606 of the rocker 600 engages the teeth 602 of the second ratchet 110. Counterclockwise rotation of the second ratchet 110 rotates the spinal fixation element 160 counterclockwise. As such, it is possible to control the rotational direction of the spinal fixation element 160 using the switches 112 and 114 of the spinal fixation element rotation instrument 100.

The rotational direction of the ratchets 108 and 110 is controlled with the first switch 112 and the second switch 114, respectively. When the first ratchet 108 is set to rotate in a first direction and the second ratchet 110 is set to rotate in a second direction opposite to the first direction, the first ratchet 108 and the second ratchet 110 may lock each other out. When the ratchets 108 and 110 are at a locked out position, the spinal fixation element 160 may no longer be rotated in the distal end 106 of the spinal fixation element rotation instrument 100.

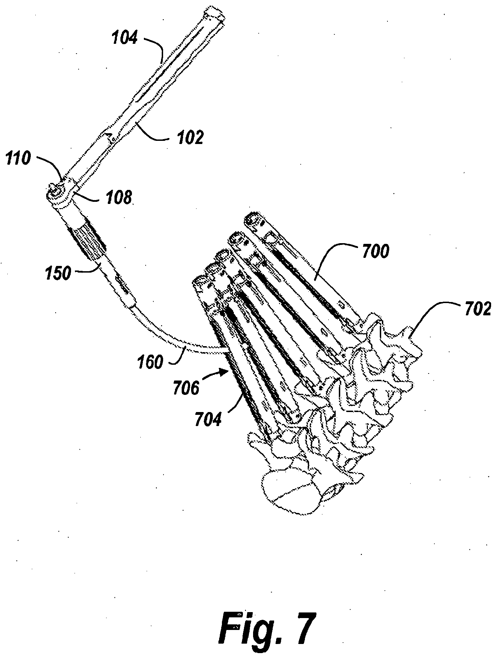

**Figure 7** illustrates an exemplary embodiment of the spinal fixation element rotation instrument 100. As illustrated in **Figure 7****,** the distal end of the spinal fixation element rotation instrument 100 couples to the proximal end 146 of the extension element 150. The proximal end 146 of the extension element 150 passes through and protrudes from the first ratchet 108 and the second ratchet 110 provided at the distal end of the spinal fixation element rotation instrument 100. The carrier 158 of the extension element 150 is coupled to the spinal fixation element 160 that will be implanted during a minimally invasive surgery to correct a spinal deformation or degeneration. A plurality of bone anchors 700 are implanted on the vertebrae 702. Each bone anchor 700 includes an opening 704 provided above the bone surface. The openings 704 of the adjacent bone anchors 700 form a passage 706. The spinal fixation element 160 is fed through the passage 706 using the spinal fixation element rotation instrument 100. If the user wants to merely advance spinal fixation element 160 in the passage 706, the user may set the lever arms 102 and 104 to rotate in opposite direction so as to put the spinal fixation element rotation instrument 100 in a locked out position. In the locked out position, the spinal fixation element rotation instrument 100 serves as a spinal fixation element holder. The spinal fixation element may be introduced through the incision using the spinal fixation element rotation instrument 100 at the locked out position. In addition, the spinal fixation element rotation instrument 100 at the locked out position may facilitate the translation of the rod from a first bone anchor to the adjacent next bone anchor. The user may still rotate the spinal fixation element 160 by rotating the spinal fixation element rotation instrument 100 similar to a spinal fixation element holder. This will allow the user to move the spinal fixation element 160 in the surgical site as necessary. However, when it is necessary to controllably rotate the spinal fixation element 160 in the passage 706, the first and second lever arms 102 and 104 of the spinal fixation element rotation instrument 100 may be set to rotate in the same direction so as to rotate the spinal fixation element 160.

If the user wants to rotate the spinal fixation element 160 clockwise, the driver arm, e.g. the second lever arm 104, is rotated clockwise while the holder, e.g. the first lever arm 102, is held stationary. The clockwise rotation of the driver arm rotates the second ratchet 110 clockwise. The extension element 150 and the spinal fixation element 160 are provided in the socket of the second ratchet 110. The clockwise rotation of the second ratchet 110 rotates the spinal fixation element 110 clockwise. The first ratchet 108 attached to the holder arm keeps the extension element 150 and the spinal fixation element 160 from rotating back toward their initial position, i.e. rotating counterclockwise. The driver arm, e.g. the second lever arm 104, is rotated counterclockwise toward to start position to rewind the second ratchet 110. These steps are repeated for further rotating the rod clockwise.

If the user wants to rotate the spinal fixation element 160 counterclockwise, the driver arm, e.g. the second lever arm 104, is rotated counterclockwise while the holder, e.g. the first lever arm 102, is held stationary. The counterclockwise rotation of the driver arm rotates the second ratchet 110 counterclockwise. The extension element 150 and the spinal fixation element 160 are provided in the socket of the second ratchet 110. The counterclockwise rotation of the second ratchet 110 rotates the spinal fixation element 160 counterclockwise. The first ratchet 108 attached to the holder arm keeps the extension element 150 and the spinal fixation element 160 from rotating back toward their initial position, i.e. rotating clockwise. The driver arm, e.g. the second lever arm 104, is rotated clockwise toward to start position to rewind the second ratchet 110. These steps are repeated for further rotating the rod counterclockwise.

Using a ratchet with the holder arm results in improved control over the extension element 150 and the spinal fixation element 160. However, according to various embodiments of the present invention, the holder arm may be provided with other mechanisms, such as a strap wrench, instead of a ratchet to interface with the extension element 150. The holder arm is configured to keep the spinal fixation element 160 or the extension element 150 coupled to the spinal fixation element rotation instrument 100 in place. One of ordinary skill in the art will appreciate that other mechanisms, such as a strap wrench or a rotation mechanism may be provided at a distal end of the driver arm.

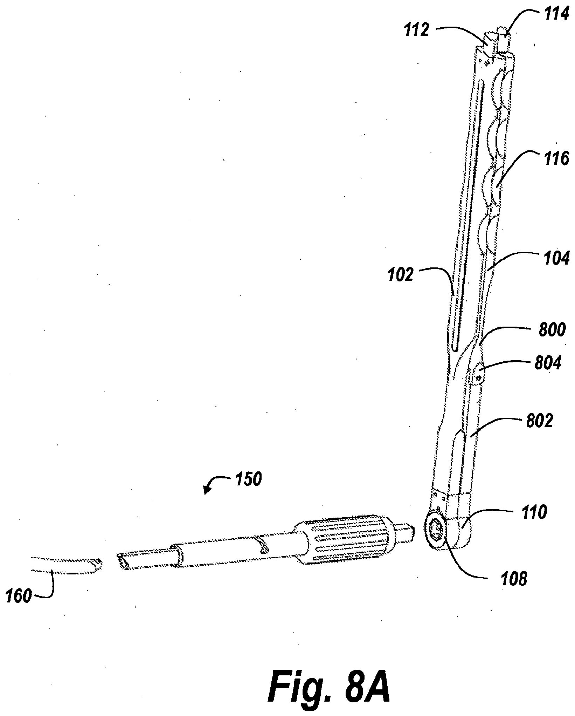

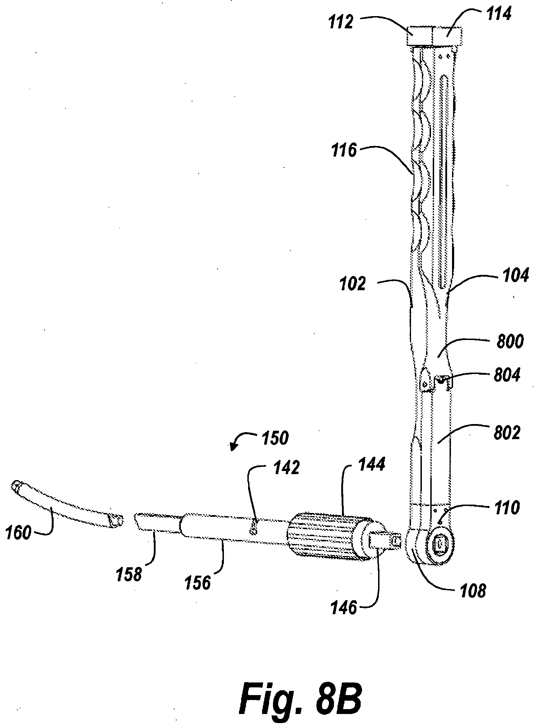

**Figures 8A-8C** illustrate another exemplary embodiment of the spinal fixation element extension instrument 100. As illustrated in **Figures 8A-8C,** the driver arm, e.g. the second lever arm 104, may include a first section 800 and a second section 802 that are coupled together using an attachment mechanism 804 such as a hinge or a loaded spring mechanism. The first section 800 may be rotated about a central axis of the attachment mechanism 804 so as to be positioned at an angle relative to the longitudinal axis of the second section 802. At the angled position, the first section 800 may serve as a handle for rotating the second ratchet 110 about the central axis thereof.

Alternatively, the driver arm, e.g. the second lever arm 104 may be a monolithic element. A handle may be attached to the second lever arm 104 for easily rotating the second lever arm 104. The handle may be fixed to the second lever arm 104. Alternatively, the handle may be attached to the second lever arm 104 using an attachment mechanism such as a hinge so as to be positioned at various angles relative to the second lever arm 104. The handle may enable the user to better maneuver the spinal fixation element rotation instrument 100.

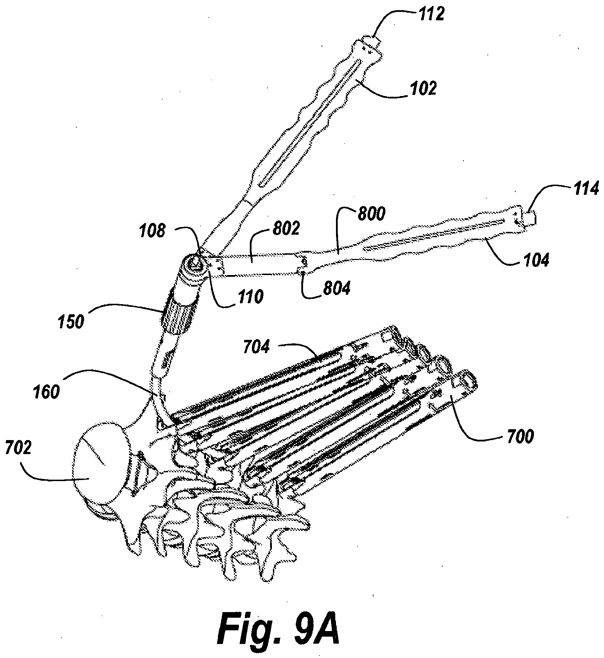

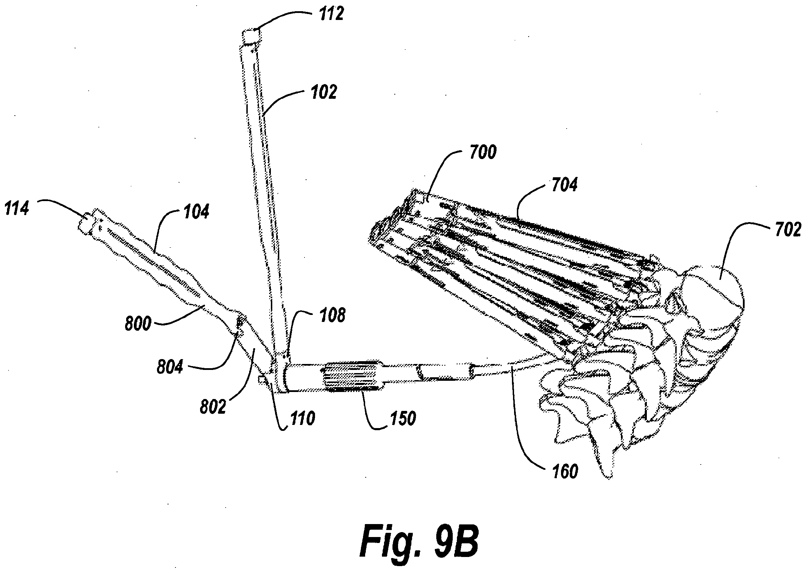

**Figures 9A** **and** **9B** illustrate an exemplary embodiment of the spinal fixation element extension instrument 100 where the first section 800 of the second lever arm 104 is provided at an angle about 15° relative to the second section 802. The first section 800 may be rotated about the central axis of the attachment mechanism 804 so as to form an angle about 0° - 45°, and preferably about 0° - 15 °, with the longitudinal axis of the second section 802. However, the positioning of the first section 800 is not limited to the angle illustrated in the figures. The first section 800 may be positioned at various angles relative to the longitudinal axis of the second section 802 to better suit the conditions of the procedure.

In the exemplary embodiment illustrated in **Figures 9A** **and** **9B****,** the first lever arm 102, i.e. the holder, is held stationary while the second lever arm 104, i.e. the driver, is rotated about the central axis of the first ratchet 108 and the second ratchet 110 using the first section 800 of the second lever arm 104. The attachment mechanism 804 holds the first section 800 in place relative to the second section 802. With the rotation of the second ratchet 110, the extension element 150 and the spinal fixation element 160 are rotated.

**Figures 9A** **and** **9B** illustrate the spinal fixation element 160 placed through the first three bone anchors 700. While the second ratchet 110 enables the spinal fixation element 160 to rotate in a first direction, the first ratchet 108 keeps the extension element 150 from rotating back toward its initial position in a second direction opposite to the first direction. Thus, the first ratchet 108 prevents the spinal fixation element 160 from inadvertently rotating in the second direction when the spinal fixation element 160 is in the passage 706. However, if the spinal fixation element 160 is not placed as desired, the surgeon may retract the spinal fixation element 160 in the passage 706 using the spinal fixation element rotation instrument 100 as a spinal fixation element holding tool. The surgeon may rotate the spinal fixation element 160 as many times as necessary using the spinal fixation element rotation instrument 100 to place the spinal fixation element 160 in the desired position.

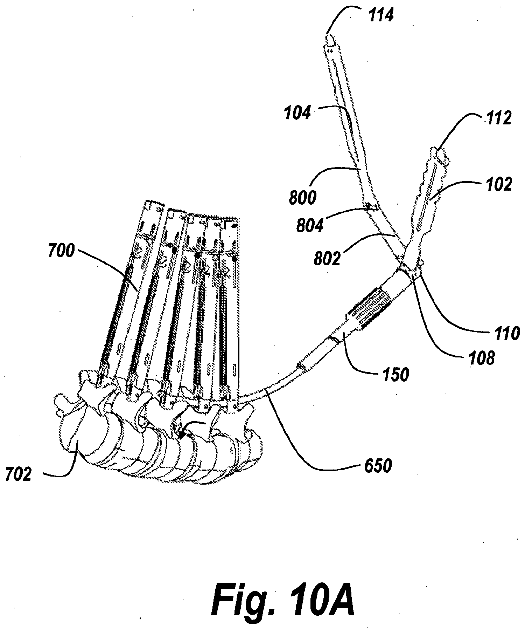

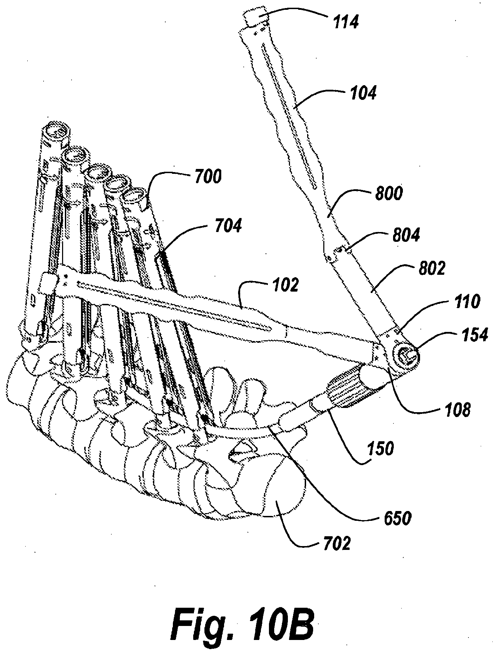

The spinal fixation element rotation instrument 100 of the present invention may be used with a variety of spinal fixation elements. A type of spinal fixation element is a curved spinal fixation element 650 illustrated in **Figures 10A** **and** **10B****.** Due to the curvature of the curved spinal fixation element 650 or the curvature of the spine at the target location, it may be preferable to insert the curved spinal fixation element 650 through the skin incision in a first position, such as a concave position. Once the curved spinal fixation element 650 is placed through the openings 704 of one or more bone anchors 700, the curved spinal fixation element 650 may be rotated to an inverted position, such as a convex position, using the spinal fixation element rotation instrument 100. The curved spinal fixation element 650 may be rotated under the skin as many times as necessary to accommodate the spinal curvatures.

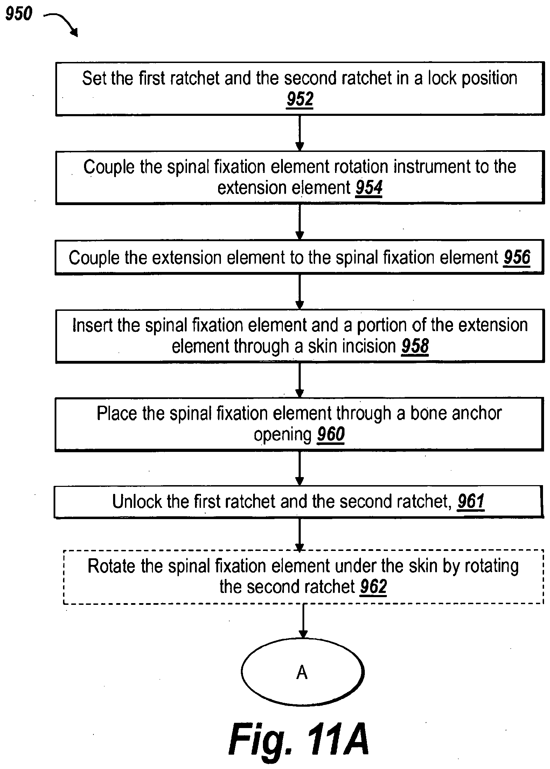

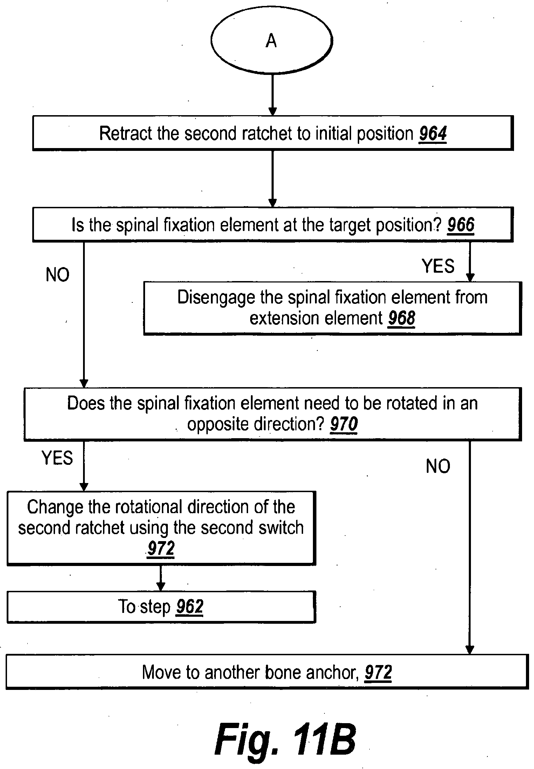

**Figures 11A** **and** **11B** illustrate a flowchart 950 of steps for placing a spinal fixation element through a plurality of bone anchors. Each bone anchor has an opening for receiving the spinal fixation element. The user first may set the first ratchet and the second ratchet of the spinal fixation element rotation instrument in a lock position. For example, the first ratchet and the second ratchet of the spinal fixation element rotation instrument may be set to rotate clockwise and counterclockwise, respectively, or vice versa (step 952). According to the embodiments of the present invention, when the ratchets are set to rotate in opposite directions, the ratchets lock each other out.

The spinal fixation element rotation instrument is then coupled to the extension element (step 954). The extension element may already be coupled to the spinal fixation element. Alternatively, the extension element may be coupled to the spinal fixation element after the spinal fixation element rotation instrument is coupled to the extension element (step 956). The spinal fixation element and a portion of the extension element are inserted through a skin incision (step 958).

During the minimally invasive surgeries, it is possible to insert the spinal fixation element and a portion of the extension element through a small skin incision without inserting the spinal fixation element rotation instrument through the skin incision, thus reducing the trauma to the tissue at the surgery site. Once under the skin, the spinal fixation element is placed through a bone anchor opening (step 960). The user then sets the first ratchet and the second ratchet of the spinal fixation element rotation instrument in an unlock position. The first ratchet and the second ratchet of the spinal fixation element rotation instrument may be set to rotate clockwise or counterclockwise (step 961). The rotational directional of the ratchets are configured to be in the unlock position when they are set to rotate in the same direction. If it is necessary to rotate the spinal fixation element under the skin, the second ratchet may be rotated clockwise from an initial position to rotate the extension element and the spinal fixation element clockwise while the first ratchet is held stationary (step 962). Step 962 may be repeated as many times as necessary during the operation. The second ratchet is then retracted to the initial position, i.e. the second ratchet is rewound (step 964). If the spinal fixation element is at a target position (step 966), i.e. placed through the openings of the bone anchors and suitably placed under the skin, the spinal fixation element is disengaged from the extension element (step 968). The extension element carrying the spinal fixation element may be equipped with a decoupling mechanism for controllably decoupling the spinal fixation element from the extension element. The spinal fixation element is implanted under the skin, in the patient while the extension element is removed.

If the spinal fixation element is not at a target position, the user returns to step 960 and repeats the above discussed steps to position the spinal fixation element through the openings of the bone anchors. It may be necessary to rotate the spinal fixation element in an opposite direction, e.g. counterclockwise, for better placement (step 970). The user may then change the rotational direction of the first ratchet and the second ratchet using the first switch and the second switch provided at the proximal end of the first lever arm and the second lever arm, respectively, (step 972) and return to step 962 to repeat the above discussed steps to position the spinal fixation element. Since the rotational direction of the second ratchet is changed, counterclockwise rotation of the first ratchet and the second ratchet rotate the spinal fixation element counterclockwise. When it is not necessary to rotate the spinal fixation element in an opposite direction in step 970, the fixation element may proceed to another bone anchor (974). The spinal fixation element is disengaged from the extension element once the spinal fixation element is placed in a desired position.

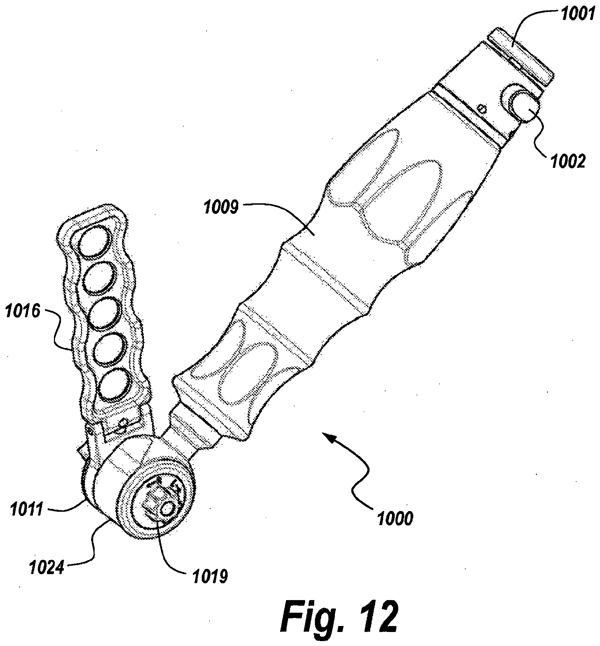

**Figure 12** illustrates an exemplary spinal fixation element rotation instrument 1000 according to another embodiment of the present invention. The spinal fixation element rotation instrument 1000 illustrated in **Figure 12** includes a handle 1009 and a side handle 1016 corresponding to the second lever arm 104 and the first lever arm 102, respectively, of the spinal fixation element rotation instrument 100 illustrated in **Figure 1A****.** Similar to the first lever arm 102 and the second lever arm 104 in the spinal fixation element rotation instrument 100, the side handle 1016 may be held stationary while the handle 1009 is configured to rotate in a predetermined direction so that the handle 1009 rotates a spinal fixation element coupled to the spinal fixation element rotation instrument 1000 from an initial position to a rotated position. One of ordinary skill in the art will appreciate that the handle 1009 may be held stationary while the side handle 1016 is configured to rotate in a predetermined direction in other embodiments.

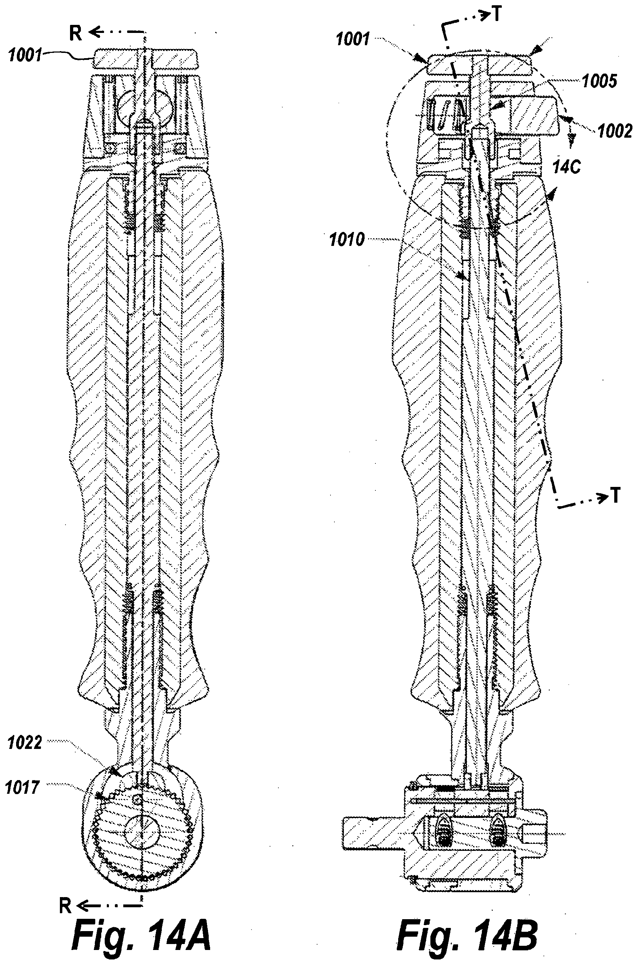

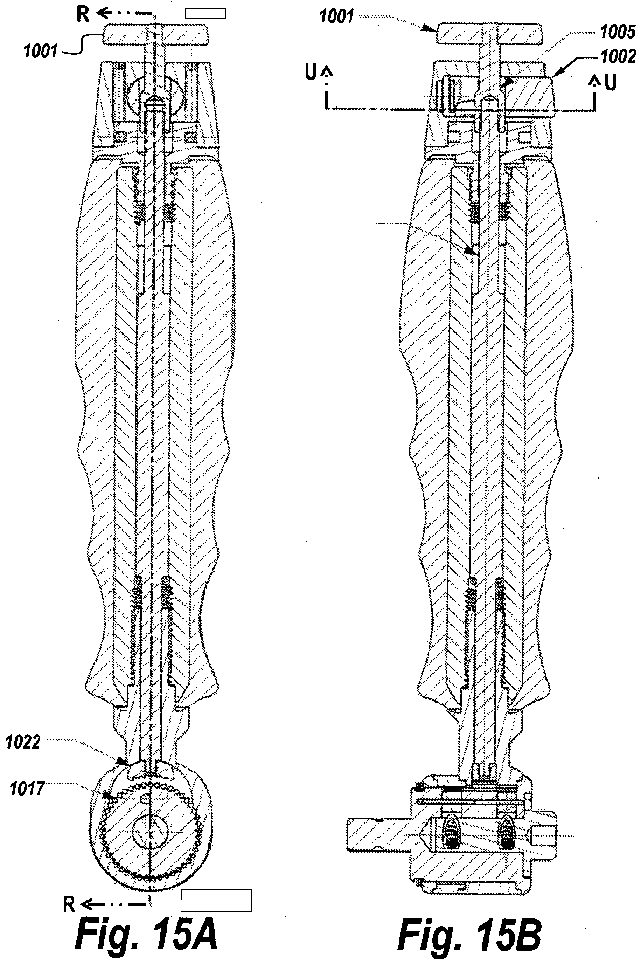

A locking button 1001 may be provided on the top of the handle 1009. When the locking button 1001 is depressed, the spinal fixation element rotation instrument 1000 is configured to be in a locked position where the spinal fixation element is prevented from rotating. A release button 1002 may also be provided on the top of the handle 1009. When the release button 1002 is depressed, the locking button 1001 returns to its initial position and the spinal fixation element rotation instrument 1000 is configured to be in an unlocked position where the spinal fixation element is allowed to rotate by rotating the side handle. One of ordinary skill in the art will appreciate that the locations of the locking button and the release button are illustrative and the locking button and the release button may be provided at different locations in other embodiments. The locked position of the spinal fixation element rotation instrument 1000 will be described below with reference to **Figures 14A-14D****.** The unlocked position of the spinal fixation element rotation instrument 1000 will also be described below with reference to **Figures 15A-15C**.

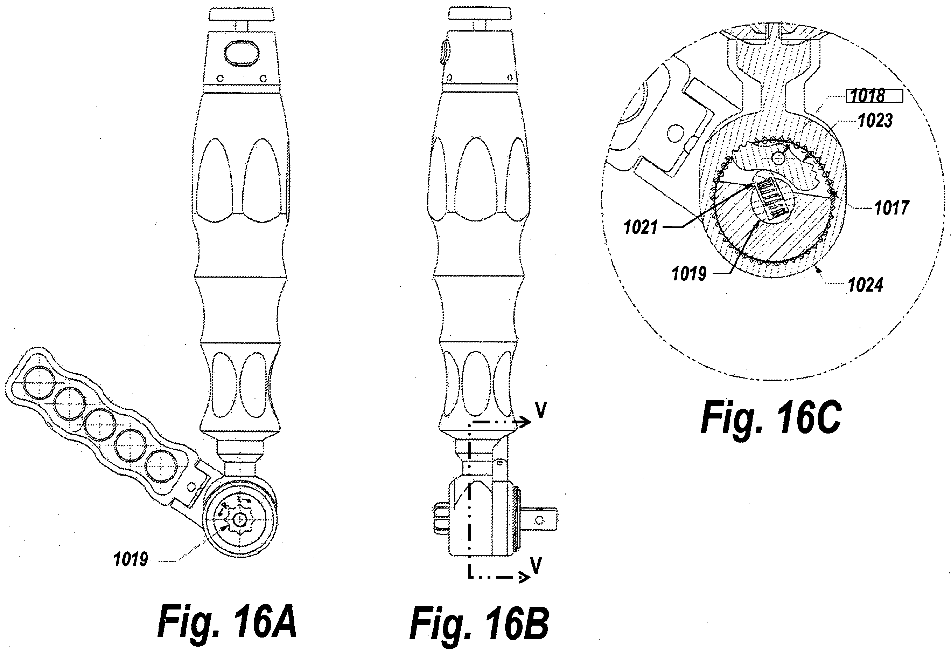

The handle 1009 is coupled to a handle housing 1024 where a first ratchet mechanism is provided so that the handle 1009 and the handle housing 1024 rotate together. The side handle 1016 is coupled to a front housing 1011 where a second ratchet mechanism is provided so that the side handle 1016 and the front housing 1011 rotate together. The handle housing 1024 and the front housing 1011 are coupled to each other. A direction switch 1019 may be provided on the handle housing 1024 to control the rotational direction of a spinal fixation element when the front housing 1011 and the side handle 1016 rotate. The first ratchet mechanism and the second ratchet mechanism and the direction switch will be described below with reference to **Figures 16A-16C**.

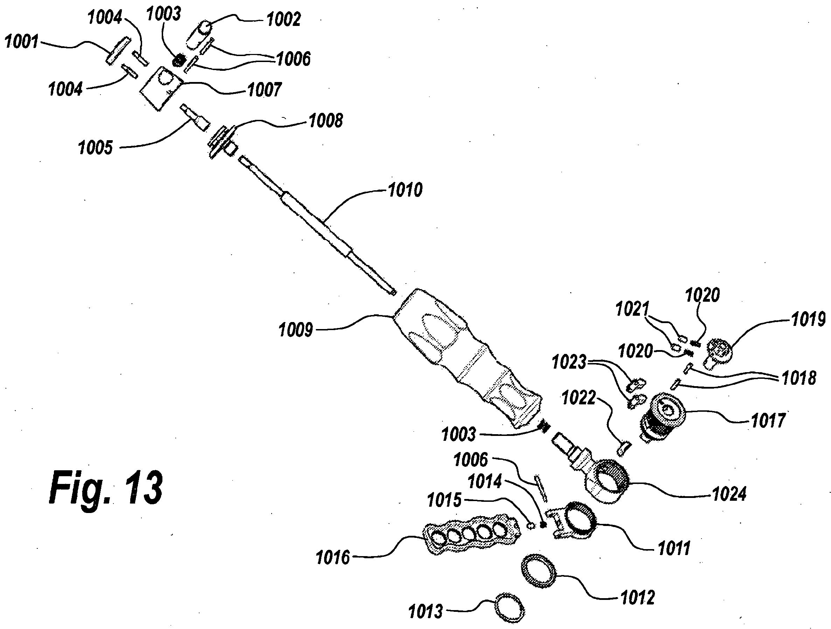

**Figure 13** illustrates an exploded view of the exemplary spinal fixation element rotation instrument 1000 illustrated in **Figure 12****.** As illustrated in **Figure 13****,** a lower locking shaft 1010 is inserted through the handle 1009. The lower locking shaft 1010 is coupled to a stop shoe 1022, which is disposed within the handle housing 1024. A handle adaptor 1008 is disposed on the upper end of the handle 1009. The lower locking shaft 1010 passes through the handle adaptor 1008. An upper locking shaft 1005 is disposed on the upper surface of the handle adaptor 1008. The upper locking shaft 1005 includes portions with a large diameter and a small diameter. The large diameter portion of the upper locking shaft 1005 is coupled to the lower locking shaft 1010. A handle cap 1007 is coupled to the handle adaptor 1008 using pins 1006 and set screws 1004. The release button 1002 is inserted through the side of the handle cap 1007 with a spring 1003. The locking button 1001 is coupled to the small diameter portion of the upper locking shaft 1005.

The side handle 1016 is coupled to the front housing 1011 using a pin 1006, a spring 1014, and a detent pin 1015. The side handle 1016 may be rotated about the pin 1006 so as to be positioned at an angle relative to the front housing 1011.

A carrier shaft 1017 is inserted into the handle housing 1024 and the front housing 1011. The teeth on the carrier shaft 1017 may engage the stop shoe 1022 when the locking button is depressed. A retaining cap 1012 and a ring 1013 are coupled to the front housing 1011.

The direction switch 1019 is inserted in the carrier shaft 1017. Pivot pins 1018 are also inserted in the carrier shaft 1017 so that rocker pawls 1023 rotate about the pivot pins 1018. Springs 1020 and spring caps 1021 are disposed between the direction switch 1019 and the rocker pawls 1023 so that the direction switch 1019 control the rotation of the rocker pawls 1023 using the springs 1020 and the spring caps 1021. The rocker pawls 1023 engage the handle housing 1024 and the front housing 1011.

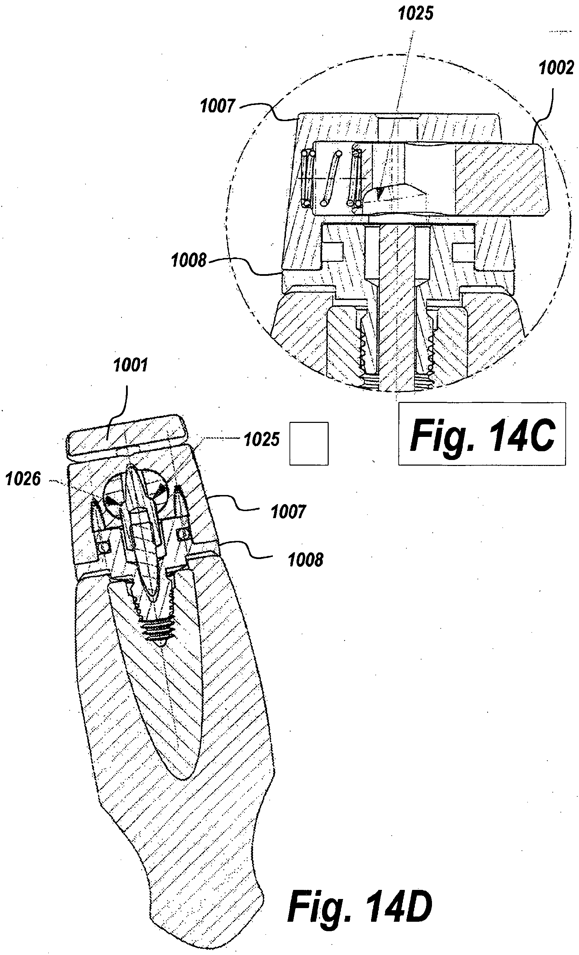

**Figures 14A-14D** illustrate the locked position of the exemplary spinal fixation element rotation instrument 1000 illustrated **in** **Figures 12** **and** **13****.** **Figure 14A** illustrates a cross-sectional view of the combined structure of the components depicted in **Figure 13****.** As illustrated in **Figure 14A**, when the locking button 1001 is depressed, the stop shoe 1022 moves down so that the stop shoe 1022 engages the teeth on the carrier shaft 1017. Since the carrier shaft 1017 engages the stop shoe 1022, the rotation of the carrier shaft 1017 is prevented. **Figure 14B** illustrates a cross-sectional view of the exemplary spinal fixation element rotation instrument 1000 along the R-R line depicted in **Figure 14A****.** When the locking button 1001 is depressed, it allows the release button 1002 to extend out. The radiused ramp on the release button 1002 engages the radiused shoulder on the upper locking shaft 1005. The engagement of the release button 1002 with the upper locking shaft 1005 forces the upper locking shaft 1005, the lower locking shaft 1010 and the stop shoe 1022 to move down. **Figure 14C** illustrates the radiused ramp 1025 on the release button 1002. The locking button and the upper locking shaft are not shown in this figure to show the radiused ramp 1025 on the release button. **Figure 14D** illustrates a cross-sectional view of the exemplary spinal fixation element rotation instrument 1000 along the T-T line depicted in **Figure 14B**. **Figure 14D** illustrates the radiused ramp 1025 on the release button 1002 and the radiused shoulder 1026 on the upper locking shaft 1005.

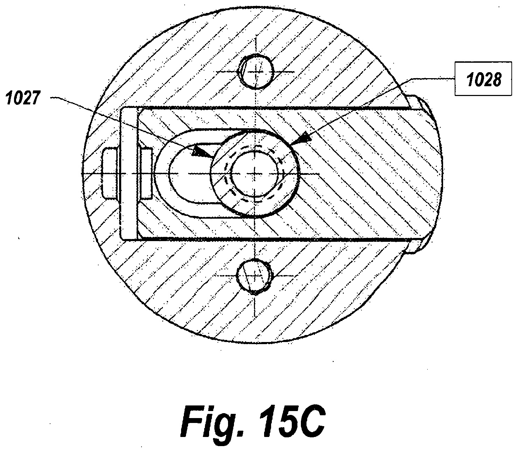

**Figures 15A-15C** illustrate the unlocked position of the exemplary spinal fixation element rotation instrument 1000 illustrated in **Figures 12** **and** **13****.** **Figure 15A** illustrates a cross-sectional view of the combined structure of the components depicted in **Figure 13****.** As illustrated in **Figure 15A****,** when the release button 1002 is depressed, the stop shoe 1022 moves up so that the stop shoe 1022 disengages from the teeth on the carrier shaft 1017. Since the carrier shaft 1017 disengages from the stop shoe 1022, the rotation of the carrier shaft 1017 is permitted. **Figure 15B** illustrates a cross-sectional view of the exemplary spinal fixation element rotation instrument 1000 along the R-R line depicted in **Figure 15A****.** When the release button 1002 is depressed, it allows the clearance bore in the release button 1002 to align with the large diameter portion of the upper locking shaft 1005. Therefore, the large diameter portion of the upper locking shaft 1005 passes through the clearance bore in the release button 1002. The upper locking shaft 1005, the lower locking shaft 1010 and the stop shoe 1022 move up to the initial position. **Figure 15C** illustrates the clearance bore 1027 in the release button 1002 and the large diameter portion 1027 of the upper locking shaft 1005.

**Figures 16A-16C** illustrate the ratchet mechanism of the exemplary spinal fixation element rotation instrument 1000 illustrated in **Figures 12** **and** **13****.** **Figure 16A** illustrates a front view of the exemplary spinal fixation element rotation instrument 1000 depicted in **Figures 12** **and** **13****.** In the front view, the direction switch 1019 faces the front. **Figure 16B** illustrates a side view of the exemplary spinal fixation element rotation instrument 1000 depicted in **Figure 16A. Figure 16C** illustrates a cross-sectional view of the exemplary spinal fixation element rotation instrument 1000 along the V-V line depicted in **Figure 16B****.** This figure shows the ratchet mechanism provided in the handle housing 1024. When the handle housing 1024 is rotated counterclockwise, the rocker pawl 1023 rotates about the pivot pin 1018. The rocker pawl 1023 moves away from the teeth in the handle housing 1024 and bears down on the spring cap 1021 which slides into the direction switch 1019 and compresses the spring 1020. To reverse the direction of the ratchet, the direction switch 1019 is rotated clockwise, causing the spring cap 1021 to contact the side of the rocker pawl 1023 on the other side of the pivot pin 1018. Those of ordinary skill in the art will appreciate that the ratchet mechanism provided in the front housing 1011 may have a like configuration.

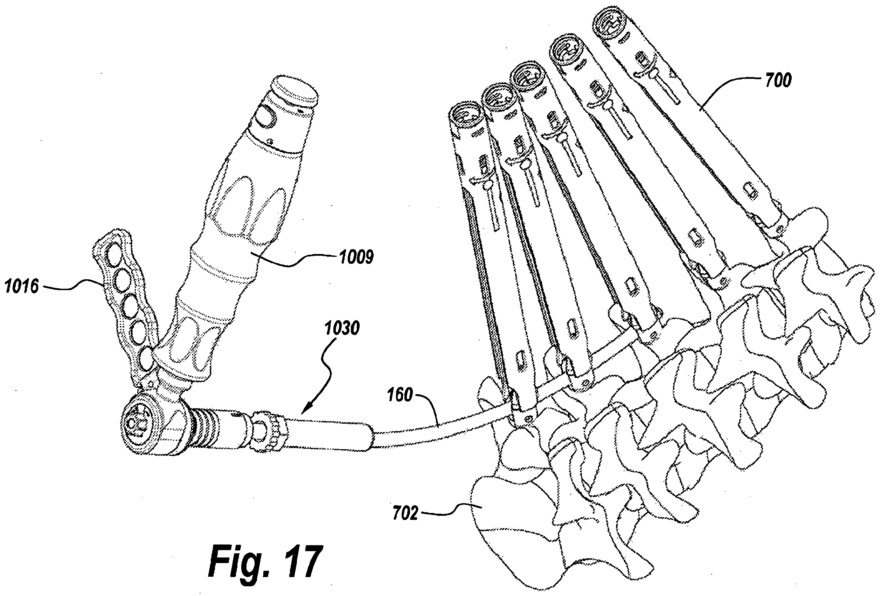

**Figure 17** illustrates the exemplary spinal fixation element rotation instrument illustrated in **Figures 12** **and** **13****,** which is coupled to an exemplary extension element 1030. The extension element 1030 is coupled to an exemplary spinal fixation element 160. In the exemplary embodiment illustrated in **Figure 17****,** the side handle 1016 is held stationary while the handle 1009 is rotated. With the rotation of the handle 1009, the extension element 1030 and the spinal fixation element 160 are rotated. The spinal fixation element 160 may be placed through the bone anchors 700. While the spinal fixation element rotation instrument 1000 is in a lock position, the spinal fixation element 160 is prevented from inadvertently rotating when the spinal fixation element 160 is inserted through the passage in the bone anchor 700. The spinal fixation element 160 may be rotated as many times as necessary using the spinal fixation element rotation instrument 1000 to place the spinal fixation element 160 in the desired position.

The extension element 1030 may include a plurality of markers (not shown) to illustrate how much the extension element 1030 has rotated. For example, each marker 1031 may indicate that the extension element has rotated 90°, 180°, 270°, 360°, etc.

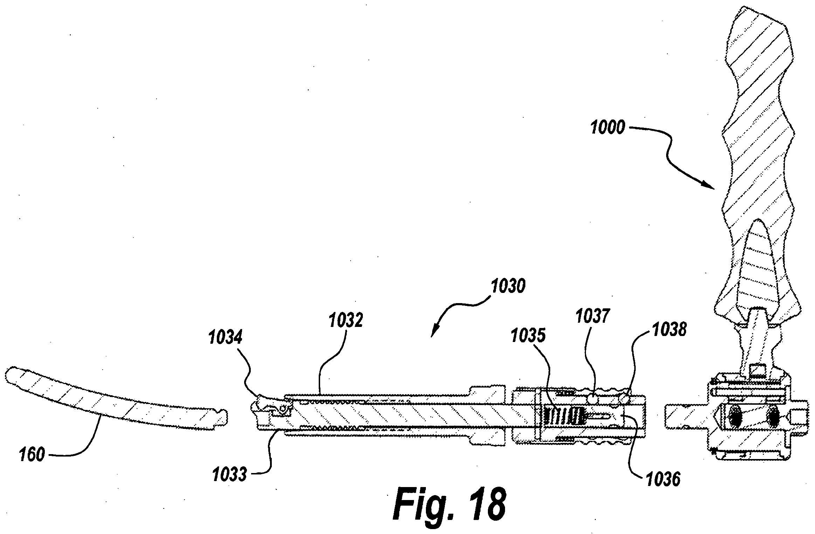

**Figure 18** illustrates a cross-sectional view of the exemplary spinal fixation element rotation instrument 1000, the exemplary extension element 1030 and the exemplary spinal fixation element 160 depicted in **Figure 17**. As illustrated in **Figure 18****,** the extension element 1030 includes a sleeve 1032 provided around a carrier 1033. The carrier 1033 is adapted to couple to the spinal fixation element 160. The carrier 1033 includes a coupling feature for tightly holding the spinal fixation element 160. For example, the spinal fixation element 160 may be coupled to the extension element 1030 via a pivoting lever with a tooth 1034. The sleeve 1032 may be slid over the carrier 1033 so that the location where the carrier 1033 engages the spinal fixation element 160 is covered by the sleeve 1032 to prevent inadvertent decoupling of the spinal fixation element 160 from the carrier 1033.

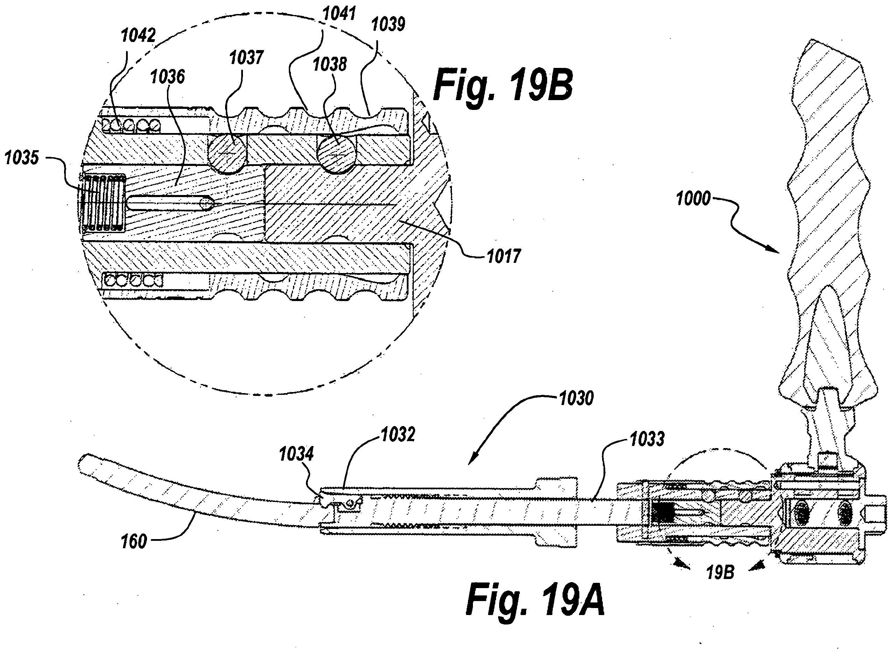

The extension element 1030 may also include a coupling mechanism for coupling the extension element 1030 to the spinal fixation element rotation instrument 1000. The coupling mechanism may include a loaded spring 1035 coupled to a moving element 1036. Balls 1037 and 1038 are provided to fall into the groove provided on the surface of the moving element 1036 and the groove provided on the surface of the carrier shaft 1017 when the spinal fixation element rotation instrument 1000 is coupled to the extension element 1030. The coupling mechanism will be described below with reference to **Figure 19B**. One of ordinary skill in the art will appreciate that the coupling mechanism described above is for illustrative purposes only and similar mechanisms may be used to couple the extension element 1030 to the spinal fixation element rotation instrument 1000.

**Figures 19A and 19B** illustrate another cross-sectional view of the exemplary spinal fixation element rotation instrument 1000, exemplary extension element 1030 and exemplary spinal fixation element 160 illustrated in **Figure 17** where the exemplary spinal fixation element rotation instrument 1000 is coupled to the exemplary extension element 1030 and the exemplary extension element 1030 is coupled to the exemplary spinal fixation element 160. As illustrated in **Figure 19A****,** the carrier 1033 is coupled to the spinal fixation element 160 through the tooth 1034. The sleeve 1032 is slid over the carrier 1033 to protect the area where the carrier 1033 engages the spinal fixation element 160. **Figure 19B** illustrates an enlarged view of the coupling mechanism where the exemplary spinal fixation element rotation instrument 1000 is coupled to the exemplary extension element 1030. As illustrated in **Figure 19B**, the loaded spring 1035 is compressed when the carrier shaft 1017 of the spinal fixation element rotation instrument 1000 is coupled to the extension element 1030. The moving element 1036 moves toward the spring 1035 until the first ball 1037 falls into the groove provided on the surface of the moving element 1036. At that point, the outer sleeve 1041 moves forward by the spring 1042 forcing the second ball 1038 to fall into the groove provided on the surface of the carrier shaft 1017 in the spinal fixation element rotation instrument 1000. When the balls 1037 and 1038 are in the grooves, the extension element 1030 and the spinal fixation element rotation instrument 1000 are locked to each other. The outer surface of the extension element 1030 where the spinal fixation element rotation instrument 1000 is coupled may be provided with grooves 1039 to improve the user's grip on the extension element 1030 and to facilitate coupling of the extension element 1030 to the spinal fixation element rotation instrument 1000.

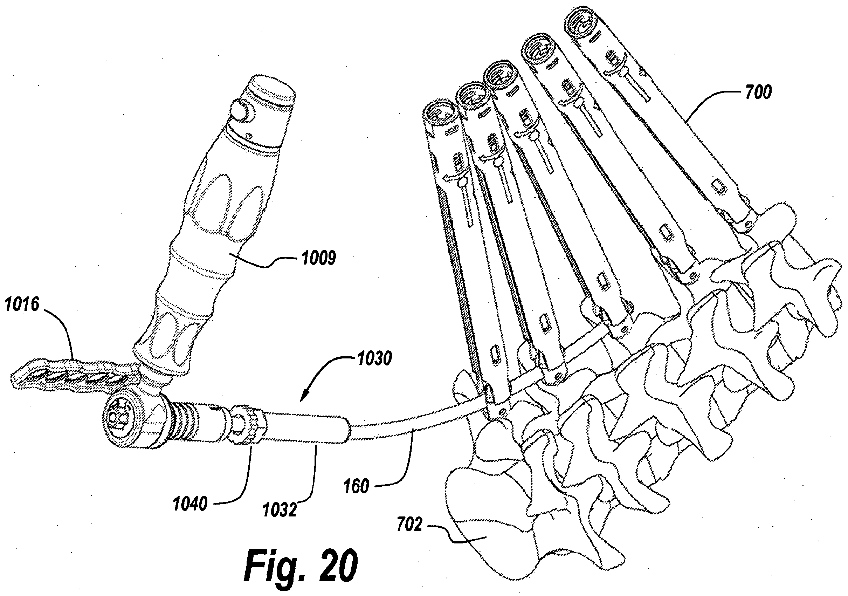

**FIG 20** illustrates another configuration of the exemplary spinal fixation element rotation instrument, the exemplary extension element and the exemplary spinal fixation element illustrated in **Figure 17****.** In **Figure 20****,** the side handle 1016 is provided at an angle about 90° relative to the front housing 1011. One of ordinary skill in the art will appreciate that the positioning of the side handle 1016 is not limited to the angle illustrated in this figure. The side handle 1016 may be positioned at various angles relative to the front housing 1011 to better suit the conditions of the procedure.

The sleeve 1032 may include an external surface feature 1040 for improving the user's grip on the sleeve 1032 to facilitate rotating of the sleeve 1032 over the carrier 1033. The external surface feature 1040 may mate with an instrument, such as a hex wrench. When the spinal fixation element 160 is in its final place, the extension element 1030 needs to be removed. However, it may be difficult for the surgeon to detach the spinal fixation element 160 from the extension element 1030. In this case, the instrument that mates with the external surface feature 1040 of the sleeve 1032 may be used to detach the spinal fixation element 160 from the extension element 1030.

While the exemplary spinal fixation element rotation instrument disclosed herein have been particularly shown and described with reference to minimally invasive surgeries, those of ordinary skill in the art will appreciate that the spinal fixation element rotation instrument described herein may also be used with an invasive, open surgery. When used in an open surgery, the spinal fixation element rotation instrument eliminates the need for secondary instruments to handle the spinal fixation element. The spinal fixation element rotation instrument according to the present invention will also eliminate the need to un-grip and re-grip the spinal fixation element between rotations during an open surgery. Hence, the spinal fixation element rotation instrument described herein may reduce the surgery time and reduce the required tools used during an open surgery.

The spinal fixation element rotation instrument described herein may be constructed of any biocompatible material including, for example, metals, such as stainless steel or titanium, polymers, ceramics, or composites thereof. The size and diameter of elements of the spinal fixation element rotation instrument may vary depending on many factors including: the type of spinal fixation elements and/or the type of extension elements used, the diameter of a surgical access port or minimally invasive incision for insertion of the spinal fixation element, the depth of the patient, the depth of the tissue surrounding the target location, etc. In an exemplary embodiment of the present invention, the length of the spinal fixation element rotation instrument may be about 200 mm. The length of the extension element may be about 150mm. The extension element may have a narrower portion that is configured to fit through the skin incision. The narrower portion of the extension element may be about 60mm. A typical skin incision fits tightly around the spinal fixation element and the narrower portion of the extension element that fits through the skin incision. The diameter of the narrower portion of the extension element may be about 10 mm. These dimensions are for illustrative purposes only and should not be viewed as limiting. One of ordinary skill in the art will appreciate that the lever arms of the spinal fixation element rotation instrument may have any dimension. However shorter lever arm will provide less mechanical advantage and thus, it will be harder to rotate the lever arm.

A person having ordinary skill in the art will appreciate that the aforementioned methods and devices for approximating bone anchors can be modified depending on the type of anchor being used, as well as the specific procedure being employed. Moreover, other devices known in the art can be used in accordance with the present invention.

One skilled in the art will appreciate further features and advantages of the invention based on the above-described embodiments. Accordingly, the invention is not to be limited by what has been particularly shown and described, except as indicated by the appended claims.

While the instruments disclosed herein have been particularly shown and described with reference to the exemplary embodiments thereof, those of ordinary skill in the art will understand that various changes may be made in the form and details herein. Those of ordinary skill in the art will recognize or be able to ascertain many equivalents to the exemplary embodiments described specifically herein by using no more than routine experimentation.

## Claims

1. A spinal fixation element rotation instrument (100) comprising:
a first lever arm (102), wherein a distal end of the first lever arm is adapted to couple to the spinal fixation element (160);
a second lever arm (104) rotatably coupled to the first lever arm at the distal end thereof, a distal end of the second lever arm being adapted to couple to the spinal fixation element, wherein the second lever arm rotates relative to the first lever arm, about a central axis of the distal ends of the first lever arm and the second lever arm to rotate the spinal fixation element from an initial position to a rotated position;
an opening provided at the distal end of the first lever arm and the second lever arm; and
an extension element (150) including a proximal end (154) adapted to couple to the instrument, the instrument receiving the extension element in a portion of the opening, and a distal end (152) adapted to couple to the spinal fixation element, the extension element including a sleeve (156) provided around a carrier (158), the carrier provided with a grabbing tip (400) for grabbing the spinal fixation element, the carrier including a coupling feature (140) for grabbing on a surface feature provided at a proximal end of the spinal fixation element:

2. The instrument of claim 1, wherein the first lever arm includes a first ratchet (108) and the second lever arm includes a second ratchet (110) forming a dual ratchet mechanism that prevents the spinal fixation element from rotating back toward the initial position.

3. The instrument of claim 1, wherein the second lever arm comprises:
a first section (800);
a second section (802); and
an attachment mechanism (804) to attach the first section to the second section so that the first section is rotatable relative to the second section about a central axis of the attachment mechanism, wherein the central axis of the attachment mechanism is perpendicular to the central axis of the distal ends of the first lever arm and the second lever arm.

4. The instrument of claim 3, wherein the attachment mechanism is a loaded spring mechanism that allows the first section to assume one or more positions relative to the second section.

5. The instrument of claim 2, further comprising:
a first switch (112) provided on a proximal end of the first lever arm, wherein the first switch allows the first ratchet to rotate clockwise when the first switch is at a first position, and the first switch allows the first ratchet to rotate counterclockwise when the first switch is at a second position.

6. The instrument of claim 5, further comprising:
a second switch (114) provided on a proximal end of the second lever arm, wherein the second switch allows the second ratchet to rotate clockwise when the second switch is at a first position, and the second switch allows the second ratchet to rotate counterclockwise when the second switch is at a second position.

7. The instrument of claim 6, wherein the spinal fixation element is rotated clockwise when the first ratchet and the second ratchet are set to rotate clockwise.

8. The instrument of claim 6, wherein the spinal fixation element is rotated counterclockwise when the first ratchet and the second ratchet are set to rotate counterclockwise.

9. The instrument of claim 2, wherein the first ratchet and the second ratchet lock each other out so as to prevent the spinal fixation element from rotating when the first ratchet is set to rotate in a first direction and the second ratchet is set to rotate in a second direction opposite to the first direction.

10. The instrument of claim 2, further comprising:
a direction switch (1019) provided on a distal end of the first lever arm, wherein the direction switch allows the first ratchet and the second ratchet to rotate clockwise when the direction switch is at a first position, and the direction switch allows the first ratchet and the second ratchet to rotate counterclockwise when the direction switch is at a second position.

11. The instrument of claim 10, further comprising:
a locking button (1001) disposed on top of the first lever arm to prevent the first ratchet and the second ratchet from rotating.

12. The instrument of claim 11, further comprising:
a release button (1002) disposed on top of the first lever arm to allow the rotation of the first ratchet and the second ratchet.

## Patentansprüche

1. Instrument (100) zum Drehen eines Wirbelsäulenfixationselements, umfassend:
einen ersten Hebelarm (102), wobei ein distales Ende des ersten Hebelarms dafür geeignet ist, an das Wirbelsäulenfixationselement (160) zu koppeln;
einen zweiten Hebelarm (104), der drehbar an das distale Ende des ersten Hebelarms gekoppelt ist, wobei ein distales Ende des zweiten Hebelarms dafür geeignet ist, an das Wirbelsäulenfixationselement zu koppeln, wobei sich der zweite Hebelarm relativ zum ersten Hebelarm um eine Mittelachse der distalen Enden des ersten Hebelarms und des zweiten Hebelarms dreht, um das Wirbelsäulenfixationselement aus einer Ausgangsstellung in eine gedrehte Stellung zu drehen;
eine Öffnung, die am distalen Ende des ersten Hebelarms und des zweiten Hebelarms vorgesehen ist; und
ein Erweiterungselement (150), das ein proximales Ende (154) umfasst, das dafür geeignet ist, an das Instrument zu koppeln, wobei das Instrument das Erweiterungselement in einem Abschnitt der Öffnung aufnimmt, und ein distales Ende (152), das dafür geeignet ist, an das Wirbelsäulenfixationselement zu koppeln, wobei das Erweiterungselement eine Hülse (156) aufweist, die rings um einen Träger (158) vorgesehen ist, der Träger mit einer Greiferspitze (400) zum Greifen des Wirbelsäulenfixationselements versehen ist und der Träger ein Kopplungsmerkmal (140) aufweist, um ein Oberflächenmerkmal zu greifen, mit dem ein proximales Ende des Wirbelsäulenfixationselements versehen ist.

2. Instrument nach Anspruch 1, wobei der erste Hebelarm eine erste Ratsche (108) aufweist und der zweite Hebelarm eine zweite Ratsche (110) aufweist, die einen Doppelratschenmechanismus bilden, der verhindert, dass sich das Wirbelsäulenfixationselement in die Ausgangsstellung zurückdreht.

3. Instrument nach Anspruch 1, wobei der zweite Hebelarm Folgendes umfasst:
einen ersten Teilabschnitt (800);
einen zweiten Teilabschnitt (802); und
einen Befestigungsmechanismus (804) zum Befestigen des ersten Teilabschnitts am zweiten Teilabschnitt, derart, dass der erste Teilabschnitt relativ zum zweiten Teilabschnitt um eine Mittelachse des Befestigungsmechanismus drehbar ist, wobei die Mittelachse des Befestigungsmechanismus senkrecht zur Mittelachse der distalen Enden des ersten Hebelarms und des zweiten Hebelarms ist.

4. Instrument nach Anspruch 3, wobei der Befestigungsmechanismus ein vorgespannter Federmechanismus ist, der ermöglicht, dass der erste Teilabschnitt eine oder mehrere Stellungen relativ zum zweiten Teilabschnitt einnimmt.

5. Instrument nach Anspruch 2, ferner umfassend:
einen ersten Schalter (112), der an einem proximalen Ende des ersten Hebelarms vorgesehen ist, wobei der erste Schalter, wenn er in einer ersten Stellung ist, ermöglicht, dass sich die erste Ratsche im Uhrzeigersinn dreht, und der erste Schalter, wenn er in einer zweiten Stellung ist, ermöglicht, dass sich die erste Ratsche gegen den Uhrzeigersinn dreht.

6. Instrument nach Anspruch 5, ferner umfassend:
einen zweiten Schalter (114), der an einem proximalen Ende des zweiten Hebelarms vorgesehen ist, wobei der zweite Schalter, wenn er in einer ersten Stellung ist, ermöglicht, dass sich die zweite Ratsche im Uhrzeigersinn dreht, und der zweite Schalter, wenn er in einer zweiten Stellung ist, ermöglicht, dass sich die zweite Ratsche gegen den Uhrzeigersinn dreht.

7. Instrument nach Anspruch 6, wobei das Wirbelsäulenfixationselement im Uhrzeigersinn gedreht wird, wenn die erste Ratsche und die zweite Ratsche für ein Drehen im Uhrzeigersinn eingestellt sind.

8. Instrument nach Anspruch 6, wobei das Wirbelsäulenfixationselement gegen den Uhrzeigersinn gedreht wird, wenn die erste Ratsche und die zweite Ratsche für ein Drehen gegen den Uhrzeigersinn eingestellt sind.

9. Instrument nach Anspruch 2, wobei die erste Ratsche und die zweite Ratsche sich gegenseitig sperren, sodass ein Drehen des Wirbelsäulenfixationselements verhindert wird, wenn die erste Ratsche für ein Drehen in eine erste Richtung eingestellt ist und die zweite Ratsche für ein Drehen in eine zweite Richtung, entgegengesetzt zur ersten Richtung, eingestellt ist.

10. Instrument nach Anspruch 2, ferner umfassend:
einen Richtungsschalter (1019), der an einem distalen Ende des ersten Hebelarms vorgesehen ist, wobei der Richtungsschalter, wenn er in einer ersten Stellung ist, ermöglicht, dass sich die erste Ratsche und die zweite Ratsche im Uhrzeigersinn drehen, und der Richtungsschalter, wenn er in einer zweiten Stellung ist, ermöglicht, dass sich die erste Ratsche und die zweite Ratsche gegen den Uhrzeigersinn drehen.

11. Instrument nach Anspruch 10, ferner umfassend:
einen oben am ersten Hebelarm angeordneten Verriegelungsknopf (1001), um ein Drehen der ersten Ratsche und der zweiten Ratsche zu verhindern.

12. Instrument nach Anspruch 11, ferner umfassend:
einen oben am ersten Hebelarm angeordneten Entriegelungsknopf (1002), um das Drehen der ersten Ratsche und der zweiten Ratsche zuzulassen.

## Revendications

1. Instrument de rotation d'élément de fixation spinale (100) comprenant :
un premier bras de levier (102), dans lequel une extrémité distale du premier bras de levier est adaptée de manière à être couplée à l'élément de fixation spinale (160) ;
un second bras de levier (104) couplé à rotation au premier bras de levier au niveau de son extrémité distale, une extrémité distale du second bras de levier étant adaptée de manière à être couplée à l'élément de fixation spinale, dans lequel le second bras de levier tourne par rapport au premier bras de levier autour d'un axe central des extrémités distales du premier bras de levier et du second bras de levier afin de faire tourner l'élément de fixation spinale depuis une position initiale jusqu'à une position tournée ;
une ouverture ménagée au niveau de l'extrémité distale du premier bras de levier et du second bras de levier ; et
un élément d'extension (150) incluant une extrémité proximale (154) adaptée de manière à être couplée à l'instrument, l'instrument recevant l'élément d'extension dans une partie de l'ouverture, et une extrémité distale (152) adaptée de manière à être couplée à l'élément de fixation spinale, l'élément d'extension incluant une gaine (156) prévue autour d'un support (158), le support étant muni d'un embout de prise (400) pour prendre l'élément de fixation spinale, le support incluant un élément de couplage (140) destiné à s'accrocher sur un élément de surface prévu au niveau d'une extrémité proximale de l'élément de fixation spinale.

2. Instrument selon la revendication 1, dans lequel le premier bras de levier inclut un premier rochet (108) et le second bras de levier inclut un second rochet (110) qui forment un mécanisme à deux rochets qui empêche que l'élément de fixation spinale n'effectue une rétro-rotation en direction de la position initiale.

3. Instrument selon la revendication 1, dans lequel le second bras de levier comprend :
une première section (800) ;
une seconde section (802) ; et
un mécanisme d'attache (804) pour attacher la première section à la seconde section de telle sorte que la première section puisse tourner par rapport à la seconde section autour d'un axe central du mécanisme d'attache, dans lequel l'axe central du mécanisme d'attache est perpendiculaire à l'axe central des extrémités distales du premier bras de levier et du second bras de levier.

4. Instrument selon la revendication 3, dans lequel le mécanisme d'attache est un mécanisme à ressort chargé qui permet que la première section prenne une ou plusieurs position(s) par rapport à la seconde section.

5. Instrument selon la revendication 2, comprenant en outre :
un premier commutateur (112) prévu sur une extrémité proximale du premier bras de levier, dans lequel le premier commutateur permet que le premier rochet tourne dans le sens des aiguilles d'une montre lorsque le premier commutateur est dans une première position, et le premier commutateur permet que le premier rochet tourne dans le sens inverse des aiguilles d'une montre lorsque le premier commutateur est dans une seconde position.

6. Instrument selon la revendication 5, comprenant en outre :
un second commutateur (114) prévu sur une extrémité proximale du second bras de levier, dans lequel le second commutateur permet que le second rochet tourne dans le sens des aiguilles d'une montre lorsque le second commutateur est dans une première position, et le second commutateur permet que le second rochet tourne dans le sens inverse des aiguilles d'une montre lorsque le second commutateur est dans une seconde position.

7. Instrument selon la revendication 6, dans lequel l'élément de fixation spinale est tourné dans le sens des aiguilles d'une montre lorsque le premier rochet et le second rochet sont placés de manière à tourner dans le sens des aiguilles d'une montre.

8. Instrument selon la revendication 6, dans lequel l'élément de fixation spinale est tourné dans le sens inverse des aiguilles d'une montre lorsque le premier rochet et le second rochet sont placés de manière à tourner dans le sens inverse des aiguilles d'une montre.

9. Instrument selon la revendication 2, dans lequel le premier rochet et le second rochet se verrouillent l'un l'autre de manière à empêcher que l'élément de fixation spinale ne tourne lorsque le premier rochet est placé de manière à tourner dans une première direction et que le second rochet est placé de manière à tourner dans une seconde direction opposée à la première direction.

10. Instrument selon la revendication 2, comprenant en outre :
un commutateur de direction (1019) prévu sur une extrémité distale du premier bras de levier, dans lequel le commutateur de direction permet que le premier rochet et le second rochet tournent dans le sens des aiguilles d'une montre lorsque le commutateur de direction est dans une première position, et le commutateur de direction permet que le premier rochet et le second rochet tournent dans le sens inverse des aiguilles d'une montre lorsque le commutateur de direction est dans une seconde position.

11. Instrument selon la revendication 10, comprenant en outre :
un bouton de blocage (1001) disposé sur le sommet du premier bras de levier de manière à empêcher la rotation du premier rochet et du second rochet.

12. Instrument selon la revendication 11, comprenant en outre :
un bouton de libération (1002) disposé sur le sommet du premier bras de levier de manière à permettre la rotation du premier rochet et du second rochet.
